# EUROPÄISCHE PATENTSCHRIFT

(11) **EP 0 900 087 B1**
(45) Veröffentlichungstag und Bekanntmachung des Hinweises auf die Patenterteilung: **09.10.2002**
(21) Anmeldenummer: 97915196.6
(22) Anmeldetag: 08.04.1997
(51) Int. Cl.: A61K 39/12, A61L 2/00

(54) **VERFAHREN ZUR DESINTEGRATION VON NUCLEINSÄUREN UND HERSTELLUNG VON QUALITÄTSGESICHERTEN BIOLOGISCHEN PRODUKTEN**
PROCESS FOR DISINTEGRATING NUCLEIC ACIDS AND PREPARING BIOLOGICAL PRODUCTS OF GUARANTEED QUALITY
PROCEDE DE DESINTEGRATION D'ACIDES NUCLEIQUES ET DE PREPARATION DE PRODUITS BIOLOGIQUES DE QUALITE GARANTIE

(30) Priorität: 09.04.1996 AT 62996
(43) Veröffentlichungstag der Anmeldung: 10.03.1999
(73) Patentinhaber: Baxter Aktiengesellschaft, 1221 Wien (AT)
(72) Erfinder: DORNER, Friedrich, A-1230 Wien (AT); BARRETT, Noel, A-3400 Klosterneuburg/Weidling (AT); EIBL, Johann, A-1180 Wien (AT)
(74) Vertreter: Alge, Daniel, Mag. Dr. rer.nat.
(86) Internationale Anmeldenummer: AT9700068
(87) Internationale Veröffentlichungsnummer: WO97037686

(56) Entgegenhaltungen:
- EP-A- 0 152 072
- EP-A- 0 196 515
- WO-A-91/03933
- WO-A-96/20592
- CA-A- 2 159 044

## Beschreibung

Die vorliegende Erfindung betrifft die Desintegration biologisch aktiver Nucleinsäuren in einem biologisch aktiven Material durch Einwirkung eines Laserstrahles auf ein - gegebenenfalls mit einer photodynamischen Substanz behandeltes - biologisches Material, und das zu einem nicht-infektiösen biologischen Produkt führt, bei welchem im wesentlichen die gesamte biologisch aktive Nucleinsäure desintegriert ist, während die biologische Integrität und Aktivität des biologischen Materials, wie Antigenität, Immunogenität, Protektivität oder enzymatischen Charakteristika, erhalten bleiben. Die Erfindung betrifft auch die Herstellung biologischer Mittel, wie Vakzine, gentechnisch erzeugter Proteinprodukte, monoklonaler Antikörper oder Blutfaktoren, bei welchen im wesentlichen die gesamte biologisch aktive Nucleinsäure desintegriert ist.

Die Verwendung kontinuierlicher Zellinien (continuous cell lines, CCL) für die Herstellung therapeutischer biologischer Produkte und die Probleme kontaminierender Nucleinsäuren in biologischen Produkten, sowie die Inaktivierung von kontaminierender Rest-DNA in solchen biologischen Mitteln ist bekannt. Während bestimmte Produkte, wie rekombinante Proteine, Vakzine oder monoklonale Antikörper, unter Verwendung von Vertebraten-Zellen wirksam hergestellt werden, sind bestimmte andere Proteinprodukte von gewöhnlich verwendeten Zellen aufgrund der niedrigen Produktionsrate der Proteine in diesen Zellen nicht leicht erhältlich. Säugerzellen, die leicht kultivierbar sind, sind von den Aufsichtsbehörden für die Herstellung gentechnisch erzeugter Proteine und deren Verwendung für therapeutische und prophylaktische Zwecke bei Menschen oder Tieren nicht zugelassen.

Es wurde vorgeschlagen, Säuger-Zellinien für die gentechnische Herstellung vieler proteinhältiger Produkte, sowie für die Herstellung von Virusvakzinen einzusetzen. Bei der Verwendung solcher Zellinien sind jedoch bestimmte Probleme zu erwarten. Viele Produkte werden von Säugerzellen nicht direkt in das Kulturmedium freigesetzt oder sekretiert. Demgemäß erfordert die Gewinnung solcher Produkte oftmals ein Aufbrechen der Zellmembranen, um diese Produkte in das Medium freizusetzen, von welchem sie danach isoliert oder gereinigt werden können. Ein solches Aufbrechen setzt jedoch auch Nucleinsäure der Zellen in das Medium frei. Besonders da viele leicht kultivierbare Zellinien transformierte tumorogene Säuger-Zellinien mit einem onkogenen Potential sind, wie MDCK-Zellen oder SK-Hep-Zellen, ist es notwendig, zu gewährleisten, daß keine aktive Nucleinsäure als Kontamination im proteinhältigen Endprodukt oder in der Vakzinpräparation vorhanden ist. Daher wurden Versuche unternommen, Zell-Nucleinsäuren, die mit einem Oncogenitätsrisiko verbunden sein könnten, zu inaktivieren.

Impfung gegen Viruserkrankungen war eines der Hauptziele der Medizin im Laufe des lezten Jahrhunderts. Obgleich wirksame Vakzine gegen eine große Anzahl von Krankheiten entwickelt wurden, bleibt die Entwicklung sicherer und wirksamer Vakzine für eine Reihe anderer Krankheiten, z.B. HIV-Infektionen, problematisch. Die Verwendung inaktivierter oder abgetöteter viraler Agentien als Vakzin ist, obwohl sie im allgemeinen sicher ist, nicht immer wirksam, wenn die immunogenen Charakteristika des Agens geändert sind. Wenn jedoch ein hoch infektiöses, virulentes Virus für die Vakzinerzeugung verwendet wird, muß sichergestellt sein, daß das Inaktivierungsverfahren dazu führt, daß die Infektiosität vollständig verloren geht.

Die Verwendung lebender, abgeschwächter viraler Agentien als Vakzine führt oft zu einer verbesserten immunologischen Reaktivität, erhöht jedoch das mit der Impfung verbundene Risiko, daß das Vakzin selbst infektiös ist. Beispielsweise kann das Virus in eine aktive, virulente Form zurückkehren, und der Organismus sich vermehren und verbreiten, was zu einer Erkrankung führt.

So muß man im allgemeinen zwischen einer verbesserten Wirksamkeit und einem größeren Maß an Sicherheit wählen, wenn man eine Wahl trifft zwischen lebenden, abgeschwächten und inaktivierten Vakzinpräparationen. Die Wahl ist besonders schwierig, wenn das Virus gegenüber Inaktivierungsprozessen resistent ist und sehr rigorose Inaktivierungsbedingungen erfordert, wodurch die Wahrscheinlichkeit steigt, daß die antigenen Charakteristika beeinträchtigt werden.

Zum Abtöten oder Inaktivieren von Viren für die Verwendung als Vakzin sind verschiedene Techniken bekannt. Zu diesen zählen die chemische oder physikalische Behandlung, z.B. Inaktivierung mit Formaldehyd, Hydroxylamin, β-Propiolacton, UV-Bestrahlung oder photodynamische Farbstoffe, z.B. Methylenblau und sichtbares Licht. Trotz dieser allgemeinen Offenbarungen ist es notwendig, die Selektivität solcher Inaktivierungsverfahren so zu verbessern, daß die viralen Proteinhüllen ihre Integrität besser beibehalten, während die Inaktivierungs/Abtötungsraten gesteigert werden.

Ein Hauptproblem inaktivierter Virus-Vakzine ist, daß wenn der Inaktivierungsprozeß der viralen Nucleinsäure nicht vollständig ist, das Risiko besteht, daß die Virus-Nucleinsäure einem zum Virus-Vakzin zugehörigen Wirt verabreicht wird und damit unter bestimmten Umständen selbst infektiös sein kann. Beispielsweise könnte HIV-RNA durch Virus- oder Zell-Enzyme revers-transkribiert werden und in das Wirts-Genom integriert werden, was zu einer HIV-Replikation führt.

Es wurden viele Versuche unternommen, Viren in einer Probe, wie Vakzinen oder Plasma, oder Zell-Nucleinsäuren zu inaktivieren, und dabei die antigenen und immunogenen Eigenschaften der Proteine in dieser Probe zu erhalten.

US 5,106,619 offenbart ein virales Vakzin mit konservierten antigenen und immunogenen Charakteristika, erzeugt durch Inaktivierung von umhülltem und nicht-umhülltem Virus mit UV-Licht in Anwesenheit von Fourocoumarin in einer nicht-oxidierenden Atmosphäre.

US 4,880,512 offenbart ein Verfahren zur Behandlung biologischer Medien, wie Blutfraktionen, gentechnisch hergestellter Proteinprodukte und Vakzinpräparationen, und die Photolyse von Nucleinsäuren in Anwesenheit von Proteinen unter Erhaltung der antigenen und immunogenen Eigenschaften der Proteine. Biologische Medien, die Tryptophan-enthaltende Proteine aufwiesen, wurden mit gepulstem Licht von verschiedener Wellenlänge und Strömung bestrahlt.

Matthews et al. (1992, Blood Cells 18:75-89) berichtete über die Inaktivierung von Viren in virusinfizierten Zellen in Kulturmedien oder in Vollblut durch Bestrahlung mit gefilterter Xenonlichtquelle und/oder einem gepulsten Farblaser in Anwesenheit eines photodynamischen Farbstoffs. Prodouz et al. (1987, Blood 70:589-592) offenbarten ein Verfahren zur Virusinaktivierung in Proben mittels UV-Bestrahlung, wobei eine Strahlung von 308 nm eines XeX1-Eximer-Lasers verwendet wurde. In WO 96/00091 werden Proben mit elektromagnetischer Strahlung, die von einem gepulsten Lasergerät mit einer Wellenlänge im Bereich von 300 bis 370 nm erzeugt werden, zur Virus-Inaktivierung bestrahlt.

Perrin et al. (1995, Biologicals 23: 207-211) verwendeten β-Propionlacton zur Inaktivierung von kontaminierender Restzell-DNA in biologischen Mitteln. Obwohl man weiß, daß dieses Agens Virer inaktiviert, ist seine Verwendung wegen der Labilität und Reaktivität dieses Agens schwer steuerbar. Es hydrolysiert rasch in wässerigen Lösungen, und es kann auch das Produkt modifizieren, z.B. durch eine Reduktion der Fc-Funktion in Immunoglobulinen.

Die Verwendung von Nucleasen zur Inaktivierung von DNA oder RNA wurde ebenfalls vorgeschlagen, doch ist bekannt, daß dies keine sehr wirkungsvolle Methode ist, da ein hoher Prozentsatz der Nucleinsäure mit Protein verbunden und daher für Nucleasen nicht zugänglich ist.

Es ist bekannt, daß lichtempfindliche Farbstoffe und insbesondere Phenothiazin-Farbstoffe, wie Methylenblau, Bengalrosa, Neutralrot oder Toluidinblau, in Anwesenheit von Licht oder UV-Licht Viren inaktivieren kann. Dies wird durch die bevorzugte Affinität der Photofarbstoffe für Nucleinsäuren bewirkt.

Die Reaktivität von Phenothiazin-Farbstoff mit Viren wird seit dem Jahr 1930 untersucht. Perdrau et al. (1933, Proc. Roy. Soc. 122:288-298) berichteten, daß ein breites Spektrum von Viren durch die Wirkung von Methylenblau in Anwesenheit von Licht inaktiviert werden kann. Es zeigte sich, daß Rabies-Virus, Influenza-Virus, Junin-Virus, Canine distemper-Virus, HIV und Herpes simplex-Virus durch Methylenblau und Licht-Bestrahlung inaktiviert wurden (Swartz et al., 1979, Proc. Soc. Exp. Biol. Med., 161:204-209; Schnipper et al., 1980, J. Clin. Invest., 65:432-438; Cobo et al., 1986, Med. Microbiol. Immunol., 175:67-69, Bachmann et al., 1995, J. Med. Virol., 47:172-178). Umhüllte Viren besitzen im allgemeinen eine gewisse inhärente Lichtempfindlichkeit, wogegen nicht-umhüllte Viren photoresistent sind (Wallis et al., 1964, Virology 23:520-527).

Die EP O 196 515 offenbart ein Verfahren zur Inaktivierung von Viren in einer therapeutischen Proteinzusammensetzung durch Einwirkung von Licht in Anwesenheit eines Photosensibilisators auf die Zusammensetzung. Bevorzugt verwendete Photosensibilisatoren waren Protoporphyrin und Chlorpromazin. Es zeigte sich, daB Viren inaktiviert werden konnten, daß aber unter den getesteten Bedingungen auch eine Inaktivierung von Faktor VIII auftritt.

US 4,181,128 offenbart ein System zur Inaktivierung von Mikroorganismen, wie Viren, Bakterien, Toxinen und Tumorzellen, durch Behandlung mit Methylenblau, Licht, Elektrizität und einer Sauerstoff:Atmosphäre.

US 4,950,665 offenbart ein Verfahren zur Verwendung von Methylenblau zur selektiven Derivatisierung von Guanosin und zur Inaktivierung von Virus- und Krebszellen in vivo.

Die Behandlung von Plasma mit Methylenblau und Licht führt zur Inaktivierung von umhüllten Viren, einschließlich HIV, HSV, VSV, und einigen nicht-umhüllten Viren, wie SV40 (Mohr et al., 1992, Ann. Hematol. 65:224-228). Obwohl man annimmt, daß Nucleinsäuren die bevorzugten Ziele der Methylenblau/Licht-Behandlung sind (Tuite et al., 1993, Photochem. Photobiol. B: Biol. 21:103-124), wurden einige Veränderungen in Capsid-Proteinen von mit Methylenblau-Licht-inaktivierten Viren, sowie ein Verlust der Aktivität von Plasmaproteinen beobachtet (Specht et al., 1994, Photochem. Photobiol. 59:506-514; Bachmann et al., 1995, J. Med.

Virol. 47:172-178, Mohr et al., 1995, Immunol. Invest. 24:73-85).

Es ist daher offensichtlich, daß ein verbessertes Verfahren zur Inaktivierung von Nucleinsäuren in biologischen Mitteln entwickelt werden muß, welches das Risiko einer Restkontaminierung aktiver Nucleinsäuren in einem biologischen Mittel verringert, während die biologische Integrität und Aktivität proteinhältiger Produkte in dieser Probe erhalten bleibt.

Es ist daher ein Ziel der vorliegenden Erfindung, ein verbessertes Verfahren zur Desintegration von Nucleinsäuren in einem biologischen Material zur.Verfügung zu stellen.

Es ist ein anderes Ziel der vorliegenden Erfindung, ein Verfahren zur Herstellung eines sicheren biologischen Materials zu schaffen, bei welchem im wesentlichen die gesamte biologisch aktive Nucleinsäure desintegriert ist, während die biologische Integrität und Aktivität des proteinhältigen Materials erhalten bleibt.

Es ist ein weiteres Ziel der vorliegenden Erfindung, sichere biologische Mittel, wie rekombinante Proteine, monoklonale Antikörper, Vakzine oder Blutderivate, zur Verfügung zu stellen.

Zur Erreichung dieser Ziele ist gemäß einem Aspekt der vorliegenden Erfindung ein Verfahren zur Desintegration jeglicher biologisch aktiver Nucleinsäuren in einem biologisch aktiven protein-hältigen Material vorgesehen, wobei einem biologischen Material eine photodynamische Substanz, ausgewählt aus der Gruppe der Phenothiazine, zugegeben wird, die Mischung einer Bestrahlung mit Laserlicht mit einer Intensität von ≤ 0,1 J/cm² pro Zyklus ausgesetzt wird, um die Nucleinsäure-Template-Aktivität im biologischen Material um mindestens einen Faktor von 7 log-Stufen bzw. dem Gehalt an Nucleinsäure auf < 100 pg/ml, vorzugsweise < 10 pg/ml, besonders bevorzugt < 1 pg/ml zu verringern, um im wesentlichen die gesamte biologisch aktive Nucleinsäure im biologischen Material zu desintegrieren, während die biologische Integrität und Aktivität des biologischen Materials erhalten bleibt.

Bevorzugterweise wird beim erfindungsgemäßen Verfahren die Inaktivierung bzw. Desintegration der Nucleinsäure durch ein Verfahren zur Quantifizierung von Nucleinsäuren kontrolliert und ein biologisches Material erhalten, dessen Gehalt an Nucleinsäure unterhalb von 100 pg, vorzugsweise von 10 pg, besonders bevorzugt von 1 pg pro Dosis bzw. maximal 1 bis 500 Kopien/ml aufweist und damit im wesentlichen frei von Nucleinsäure-Template-Aktivität ist.

Die photodynamische Substanz ist bevorzugterweise Methylenblau, Toluidinblau oder Azurblau.

Gemäß dem Verfahren der Erfindung weist das biologisch aktive Material eine biologisch aktive Substanz mit hohem Molekulargewicht, die keine biologisch aktive Nucleinsäure ist, auf.

Vorzugsweise absorbiert die photodynamische Substanz Licht im Wellenlängenbereich des Laserstrahls oder darüber, wobei die Wellenlänge des verwendeten Laserstrahls die Wellenlänge der maximalen Aktivierungsrate der photodynamischen Substanz ist. Die Wellenlänge des verwendeten Laserstrahls liegt vorzugsweise zwischen 600 und 680 nm, insbesondere zwischen 630 und 660 nm.

Es können alle bekannten Lasergeräte, etwa ein Xenon-Laser, ein He-Ne-Laser oder ein YAG-Laser, zur Durchführung des Verfahrens eingesetzt werden, wobei jedoch ein He-Ne-Laser bevorzugt ist. Dabei wird ein System eingesetzt, bei dem das biologische Material über ein Röhrchen oder eine Küvette mit einem Glasfenster geleitet wird und das Glasfenster mit einem Laserstrahl bestrahlt wird.

Die Energiedichte, die auf das biologische Material einwirkt, wird durch die eingesetzte Strahlungsintensität des Lasers in Abhängigkeit der Verweildauer der Flüssigkeit im Laserstrahlungsbereich (mWs/mm² oder mJ/mm²) bestimmt. Die Strahlungsintensität des Lasers ist dabei die Laserleistung pro Laserstrahlungsflache (mW/mm²). Die Verweildauer im Strahl ist dabei abhängig von der Geometrie des Glasfensters (Durchmesser x Länge) und der Durchflußrate (ml/min).

Die eingesetzte Laserleistung liegt vorzugsweise in einem Bereich zwischen 1 mW bis 20 mW. Abhängig von der Strahlungsintensität des Lasers und der Verweildauer des biologischen Materials im Strahlungseinfluß des Lasers sollte bei Durchführung des Verfahrens die maximale Laser-Intensität pro Zyklus < 0,1 J/cm² sein, insbesondere ≤ 0,08 J/cm². Die Laserbestrahlung erfolgt gemäß dem erfindungsgemäßen Verfahrens mit einer Energiedichte vorzugsweise in einem Bereich zwischen 0,001 J/cm² bis maximal 0,1 J/cm², besonders bevorzugt zwischen 0,006 J/cm² und 0,06 J/cm².

Wenn eine einzige photodynamische Substanz zum Material zugegeben wird, wird das Material vorzugsweise mit einem Laserstrahl einer ausgewählten Wellenlänge bestrahlt. Wenn mehr als eine photodynamische Substanz zum biologischen Material zugegeben wird, wird das Material mit verschiedenen Laserstrahl-Wellenlängen bestrahlt, wobei die gewählten Wellenlängen ausgewählt sind aus der maximalen Aktivierungsrate der verwendeten photodynamischen Substanzen. Die zugegebene photodynamische Substanz liegt vorzugsweise in einer Konzentration zwischen 0,01 µM und 50 µM vor, vorzugsweise zwischen 1 und 4 µm, insbesondere zwischen 10 µM und 35 µM. Die photodynamische Substanz ist ausgewählt aus der Gruppe der Phenothiazine, wie Methylenblau, Neutralrot, Toluidinblau, Azurblau Bengalrot. oder Derivaten davon. Die photodynamische Substanz ist gewöhnlich in einer Lösung gelöst, welche die vollständige Auflösung der Substanz bei Vereinigung mit dem biologischen Material ermöglicht. Die photodynamische Substanz kann dem biologischen Material im Dunklen zugegeben werden, und die Mischung wird dann durch ein System mit einem Fenster für die Laserstrahl-Einwirkung für eine Zeitdauer, die ausreicht, um im wesentlichen die gesamte biologisch aktive Nucleinsäure in diesem biologischen Material zu desintegrieren, gepumpt. Um die biologische Aktivität und Integrität des biologischen Mittels zu erhalten, erfolgt die Laserstrahl-Bestrahlung in einer Zeit, die ausreicht, um die gesamte biologisch aktive Nucleinsäure zu desintegrieren, ohne die biologische Integrität und Aktivität eines proteinhältigen Materials zu beeinträchtigen. Die Parameter für die Desintegration sind der Durchmesser des Laserstrahls, der Durchmesser des Fensters, durch welches das biologische Mittel bestrahlt wird, die Fließgeschwindigkeit, mit welcher die Substanz durch das Fenster gepumpt wird, die Einwirkungszeit, die Wellenlänge des Laserstrahls, gegebenenfalls die Konzentration der photodynamischen Substanz und die Konzentration der Nucleinsäure im Material. Gemäß einem Aspekt der Erfindung kann das Verfahren in einem gepufferten System in einem pH-Bereich zwischen 5 und 10 und bei einer Temperatur zwischen 4°C und 35°C, je nach der zugesetzten photodynamischen Substanz, doch unter optimalen Bedingungen zur Desintegration der Nucleinsäure durchgeführt werden, während die biologische Integrität und Aktivität des biologisch aktiven Materials erhalten bleiben. Vorzugsweise werden die für die Nucleinsäure-Desintegration gewählten Temperatur-, pH-Wert- und Ionenstärke-Bedingungen für die Gesamtdauer des Verfahrens stabil gehalten. Die für die Inaktivierung relevanten Parameter werden für jedes biologische Material optimiert.

Wenn die Laserstrahl-Einwirkung oder die photodynamische Substanz/Laserstrahl-Behandlung zur Desintegration biologisch aktiver Nucleinsäure von mikrobiologischen oder molekularbiologischen Pathogenen in Blut, Plasma, Serum, einer Zellsuspension, einer Zellschicht, einem Zellüberstand oder einer aufgebrochenen Zellpräparation verwendet wird, die aus einer infizierten, transfektierten oder transformierten Zelle stammt, können ein Stabilisator, wie Zucker, Polyalkohole, Aminosäuren, Peptide oder Carbonsäuren, ein Quencher und/oder Scavenger, wie Mannit, Glycerin, reduziertes Glutathion oder Superoxid-Dismutase, oder eine Kombination davon dem biologischen Material vor der Laserstrahl-Einwirkung oder der Zugabe einer photodynamischen Substanz zugegeben werden. Gemäß dem Verfahren können auch ein oder mehrere verschiedene Quencher und/oder Scavenger und/oder Stabilisatoren zum biologischen Material vor der Laserstrahl-Einwirkung zugegeben werden. Die Zugabe von Quenchern von photodynamischen Reaktionen vom Typ I (freie Radikale), wie Mannit, Glycerin, reduziertes Glutathion (GSH), oder Superoxid-Dismutase (SOD) können die Verwendung hoher Lichtintensitäten oder Energieintensität (> 0,1 J/cm²) und Konzentration von beispielsweise photodynamischen Substanzen, ohne erhöhte Verluste der Aktivität, Integrität, Antigenität, Immunogenität oder enzymatischen Aktivität eines biologischen Mittels gestatten. Dies ist darauf zurückzuführen, daß eine Reaktion vom Typ II (Singulett-Sauerstoff) der Hauptvermittler der Nucleinsäure-Inaktivierung ist, wobei sowohl die Typ-I- als auch die Typ-II-Reaktion zur Protein-Inaktivierung beiträgt. Dies ist besonders wichtig für die Verwendung dieser Vorgangsweise für die Inaktivierung von Viren in Vollblut, Plasma oder Plasmaprodukten und/oder Desintegration von viraler und genomischer Nucleinsäure in biologischem Material, ohne Verluste an Aktivität und Integrität spezifischer Proteine von therapeutischer Bedeutung herbeizuführen.

Unter den für die Desintegration aktiver Nucleinsäuren verwendeten Bedingungen hat es sich gezeigt, daß schon geringe Licht- bzw. Energieintensitäten zur Inaktivierung selbst der resistentesten Viren verwendet werden können, ohne die biologische Aktivität und Integrität der Blutfaktoren oder Plasmaproteine zu zerstören.

Die Desintegration von Nucleinsäuren insbesondere von nicht-umhüllten Viren und damit ihre Inaktivierung war insbesondere im Hinblick auf den bekannten Stand der Technik überraschend, da nicht-umhüllte Viren wie Poliovirus, HAV oder Parvovirus als photoresistent gelten (Wallis et al., 1964, Virology 23:520-527; Mohr et al., 1995, Immunol. Invest. 24:73-85). Durch den Einsatz einer Kombination von photoaktiver Substanz und Laserstrahl-Behandlung mit geringer Energiedichte erlaubt das erfindungsgemäße Verfahren überraschenderweise auch die Inaktivierung nicht-umhüllter Viren in einem biologischen Material, wie etwa von Plasma, oder bei der Herstellung einer Vakzine enthaltend beispielsweise inaktiviertes Poliovirus, HAV oder Parvovirus, wobei beim biologischen Material sichergestellt ist, daß durch das Inaktivierungsverfahren die Integrität und biologische Aktivität der Proteine erhalten bleibt.

Bei einer anderen bevorzugten Ausführungsform des Verfahrens wird (werden) vor der Laserstrahl-Einwirkung eine oder mehrere photodynamische Substanz(en), mindestens ein Quencher und/oder Scavenger und/oder Stabilisator zugegeben, und die Mischung wird mit einer oder mehreren verschiedenen Laserstrahl-Wellenlänge(n) bestrahlt, wobei die Laserstrahl-Wellenlänge die Wellenlänge der maximalen Aktivierungsrate der zugesetzten photodynamischen Substanz(en) ist.

Die photodynamische Substanz und der Quencher und/oder der Scavenger und/oder der Stabilisator können in einen Behälter gegeben werden, welcher das biologisch aktive Material enthält, und die Mischung aus der (den) photodynamischen Substanz(en) und dem biologisch aktiven Material wird zwecks Laserstrahl-Einwirkung durch ein System gepumpt. Bei einer bevorzugten Ausführungsform des Verfahrens wird die dem Laserstrahl ausgesetzte Mischung direkt zu einem zweiten Behälter gepumpt. Die dem Laserstrahl ausgesetzte Mischung kann in umgekehrter Richtung durch das System für mindestens eine zweite Laser-Bestrahlung zurückgepumpt werden. Bei einer anderen bevorzugten Ausführungsform des Verfahrens, kann (können) der Mischung im zweiten Behälter mindestens eine zweite Portion von einer oder mehreren photodynamischen Substanz(en) und einem Quencher und/oder einem Scavenger und/oder einem Stabilisator zugesetzt werden, und die Mischung wird durch das System in umgekehrter Richtung für mindestens eine zweite Laser-Bestrahlung zurückgepumpt. Die Zusätze können zur Mischung in einer einzigen Zugabe oder in mehreren Zugaben zugesetzt werden, wobei die Mischung zwischen den Zugaben dem Laserstrahl ausgesetzt wird, oder sie können während der gesamten Behandlungsdauer kontinuierlich zugesetzt werden. Gewöhnlich ist die Zahl der Zugaben 1. Gemäß der vorliegenden Erfindung kann dies durch Rezyklieren der Laserstrahl-behandelten Mischung aus biologischem Material und Zusätzen in einem kontinuierlichen Verfahren erfolgen. Das Verfahren kann für mehrere Laserstrahlbehandlungszyklen, vorzugsweise 5 bis 50 Zyklen, insbesondere 10 bis 30 Zyklen, wiederholt werden.

Gemäß dem Verfahren der Erfindung ist die durch das Verfahren der Erfindung desintegrierte biologisch aktive Nucleinsäure eine DNA oder RNA.

Die vorliegende Erfindung sieht ein neues Verfahren zur Desintegration biologisch aktiver Nucleinsäuren in einem biologisch aktiven Material vor. "Biologisch aktives Material" bedeutet ein Material mit biologisch aktiven proteinhältigen Produkten, die durch unerwünschte Nucleinsäure kontaminiert sind, welche entweder von einer Zelle, einer Zellturlinie, einem Virus, einem Nucleinsäurevektor, einem prokaryontischen oder eukaryontischen Pathogen oder einem Onkogen stammt. "Desintegration" einer Nucleinsäure bedeutet die Zerstörung der Nucleinsäureeinheiten und der Aktivität in Anwesenheit von Proteinen, um den Verlust der Lebensfähigkeit und Infektiosität der Nucleinsäure zu bewirken, während die Funktionalität und biologische Integrität und Aktivität des im biologischen Material vorhandenen Proteins im wesentlichen erhalten bleibt. Ein Ziel der vorliegenden Erfindung ist die Desintegration von im wesentlichen allen biologisch aktiven Nucleinsäuren, wie DNA oder RNA, einschließlich infektiöser Nucleinsäuremoleküle, die transformationsfähig sind, welche im biologischen Material vermutet werden, während die Integrität und Aktivität der Proteine im biologischen Material intakt bleiben. Bei dem nach dem Desintegrationsprozeß erhaltenen, ein biologisch aktives Protein enthaltenden Material sind im wesentlichen alle biologisch aktiven Nucleinsäuren desintegriert. "Im wesentlichen" bedeutet, daß die restliche aktive Nucleinsäure im biologischen Material unter der Nachweisgrenze einer hochempfindlichen Nucleinsäure-Amplifikationsmethode liegt.

Gemäß dem Verfahren der vorliegenden Erfindung wird die Menge der restlichen, kontaminierenden genomischen DNA und/oder viralaktiven Nucleinsäure, RNA oder DNA, nach dem Desintegrationsoder Inaktivierungsverfahren bestimmt. Diese Bestimmung ist besonders wichtig, wenn man die kontaminierende aktive Nucleinsäure in Blutprodukten, Vakzinen, oder bei der Qualitätskontrolle rekombinanter, aus Zellkulturen stammender Produkte bestimmt. Die Effizienz der Desintegration kann durch Bestimmung der restlichen Nucleinsäure mittels eines Nucleinsäure-Amplifikationsverfahrens, wie der Ligase-Kettenreaktion (ligase chain reaction, LCR), der Amplifikation auf Basis der Nucleinsäure-Sequenz (nucleic acid sequence based amplification, NASBA), der selbstständigen Sequenz-Replikation (self-sustained sequence replication, 3SR), dem Amplifikationssystem auf Transkriptionsbasis (transcriptional based amplification system, TAS) oder der Polymerase-Kettenreaktion (polymerase chain reaction, PCR) gemessen werden. Bei einer bevorzugten Ausführungsform des Verfahrens der Erfindung ist die Amplifikationsmethode PCR.

Um die Nucleinsäure in einer Probe quantitativ zu bestimmen, wird vorzugsweise LIF-PCR (Laser-induzierte Fluoreszenz-PCR) verwendet. Durch Verwendung von nicht-radioaktiv markierten Primern (vorzugsweise werden mit einem fluoreszierenden Farbstoff markierte Primer verwendet) können die PCR-Produkte mittels LIF-PCR detektiert werden. Im Falle von LIF-PCR wird die Nucleinsäure in Anwesenheit von Fluoreszenz-markierten Primern amplifiziert, und die Amplifikationsprodukte werden danach mittels PAGE getrennt. Die Amplifikation erfolgt mit Primern, die für die entsprechende Nucleinsäure des mikrobiologischen oder molekularen Pathogens oder der kontaminierenden genomischen DNA spezifisch sind.

Um die Effizienz des Inaktivierungs- und Desintegrierungsverfahrens vor der Amplifikation zu quantifizieren, wird eine bestimmte Menge einer bekannten Nucleinsäure zur Probe als interner Standard zugegeben, wobei sich die Standard-Nucleinsäure von der zu bestimmenden genomischen oder viralen Nucleinsäure in mindestens einem detektierbaren Charakteristikum unterscheidet, doch sollte sie mit Hilfe desselben Primers amplifizierbar sein.

Bis zur vorliegenden Erfindung war es nicht möglich, biologisch aktive Nucleinsäuren in einer biologischen Probe vollständig zu desintegrieren, ohne die biologische Aktivität und/oder Integrität von in der Probe vorhandenen Proteinen wesentlich zu beeinträchtigen. Es war auch nicht möglich, die Effizienz des Nucleinsäure-Desintegrationsverfahrens mittels PCR-Amplifikationsmethoden zu bestimmen und zu quantifizieren, um ein reproduzierbares System für die Herstellung von sicheren und qualitätskontrollierten biologischen Mitteln zu besitzen.

Bevorzugterweise umfaßt das biologisch aktive Material eine biologisch aktive Substanz mit hohem Molekulargewicht, die keine biologisch aktive Nucleinsäure ist. Vorzugsweise ist das biologisch aktive Material eine Lösung, welche einen Blutfaktor, ein Plasmaprotein, das aus der Fraktionierung von Blut oder Blutplasma stammt, einen Plasmafaktor, ein Immunglobulin, ein gentechnisch hergestelltes proteinhältiges Produkt, wie ein rekombinantes Protein oder einen monoklonalen Antikörper oder ein mikrobiologisches und/oder molekulares Pathogen, wie ein Virus oder ein Bakterium, oder einen Teil davon aufweist. Bei einer bevorzugten Ausführungsform des Verfahrens umfaßt das biologische Material ein proteinhältiges Produkt. Vorzugsweise ist das Material ausgewählt aus einer Lösung umfassend einen Blutfaktor, wie Faktor II, Faktor V, Faktor VII, Faktor VIII, Faktor IX, Faktor X, Faktor XI, Faktor XIII, Protein C, Protein S, von Willebrand-Faktor oder ein Plasmaprotein, wie Plasminogen, Plasminogen-Aktivator-Inhibitor, Antithrombin III, Cl-Esterase-Inhibitor, Albumin, Fibrinogen, γ-Globulin, Immunoglobulin, ein gentechnisch erzeugtes Proteinprodukt, wie ein rekombinantes Protein oder einen monoklonalen Antikörper oder einen Teil davon, oder ein Pathogen, wie ein Virus oder ein Bakterium. Das biologisch aktive Material kann Vollblut, eine Blutkomponente oder ein Derivat davon, wie eine Plasmafraktion, ein Plasmaprotein mit einer Nucleinsäure in Form eines potentiellen mikrobiologischen oder molekularen Pathogens, eine Zellsuspension, eine Zellschicht, ein Zellkulturüberstand oder eine aufgebrochene Zellpräparation sein, die von infizierten, transfektierten oder transformierten Zellen oder einer Zellkultur stammt, die eine kontaminierende biologisch aktive Nucleinsäure aufweist.

Das Verfahren der vorliegenden Erfindung ist geeignet, biologisch aktive Nucleinsäure zu desintegrieren und virale Pathogene, wie HIV-1, HIV-2, HAV, HCV, HBV, Parvovirus, CMV, HHV-6, HTLV-1, HTLV-2, EBV, HDV, Echovirus und Coxsackie-Virus in Plasma oder einer Plasmafraktion zu inaktivieren.

zellen in dieser Zellkultur, Zellsuspension, Zellschicht, Zellkulturüberstand oder in der aufgebrochenen Zellpräparation können von Vertebraten-Zellen, wie VERO-Zellen, CHO-Zellen, BHK-Zellen, Hühnerembryozellen, MDCK-Zellen, CV-1-Zellen, LLC-MK2-Zellen, MDBK-Zellen, WI-38-Zellen, MRC-5-Zellen, SR-Hep-zellen oder einer anderen kontinuierlichen Zellinie, wie einer Tumorzellinie oder einer Hybridom-Zellinie, stammen.

Die biologisch aktive Nucleinsäure, die mittels des Verfahrens der Erfindung desintegriert wird, ist DNA oder RNA. Die Nucleinsäure kann eine genomische DNA aus einem Genom oder einem Teil davon, etwa ein Gen, sein. Die intakte Nucleinsäure hat eine Nucleinsäure-Template-Aktivität und umfaßt eine Sequenz für Primer und Polymerase-Bindung. Sie ist auch mittels eines Nucleinsäure-Amplifikationsverfahrens amplifizierbar. Die Nucleinsäure kann aus einem eukaryontischen Ausgangsmaterial, wie einer Vertebraten-Zelle, einer Tumor-Zellinie oder einer Hybridom-Zelllinie, aus einem Mikroorganismus, wie einem Protozoon, einem Bakterium, einem Virus, einem mikrobiologischen oder molekularen Pathogen oder einem Teil davon, oder aus einem Onkogen oder einem Protoonkogen stammen.

Bei einer anderen bevorzugten Ausführungsform des Verfahrens stammt das virushältige biologische Material aus einer Kultur von virusinfizierten Zellen, einem Kulturüberstand von virusinfizierten Zellen, einer Zellkomponente von virusinfizierten Zellen oder einer ein gereinigtes Virus aufweisenden Lösung.

Gemäß einem anderen Aspekt der Erfindung wird das Verfahren zur Erzeugung eines inaktivierten Virus verwendet. Vorzugsweise ist das Virus ein Lipid-(umhülltes) oder Non-Lipid-(nicht umhülltes) Virus, ein DNA oder ein RNA-Virus, oder ein abgeschwächtes Virus.

Mit dem erfindungsgemäßen Verfahren kann z.B. ein inaktiviertes Virus erhalten werden, das eine desintegrierte biologisch aktive Nucleinsäure aufweist, während seine biologische Integrität und Aktivität, wie antigene, immunogene und protektive Eigenschaft, erhalten bleibt. Das inaktivierte Virus mit den oben beschriebenen Eigenschaften kann jede Art von Virus sein, wie ein Lipid-(umhülltes) oder ein Non-Lipid-(nicht-umhülltes) Virus, ein DNAoder ein RNA-Virus, oder ein abgeschwächtes Virus.

Das erfindungsgemäß inaktivierte Virus kann zu einer Vakzinpräparation gegebenenfalls mit einem physiologisch akzeptablen Träger formuliert werden.

Das erfindungsgemäß zu inaktivierende Virus ist bevorzugterweise ein Lipid-(umhülltes), ein nicht-Lipid-(umhülltes) Virus, ein DNA- oder ein RNA-Virus oder ein attenuiertes Virus.

Vorzugsweise werden mit dem erfindungsgemäßen Verfahren Viren, ausgewählt aus der Gruppe Adenovirus, Herpesvirus, Papoavirus, Poxvirus, Parvovirus, Reovirus, Retrovirus, Myxovirus, Paramyxovirus, Picornavirus, Togavirus, Flavivirus, Orthomyxovirus oder Rhadovirus, vorzugsweise ein Influenzavirus, HIV, HCV, HAV, HBV, TBEV oder CMV ist, wobei das Virus gegebenenfalls attenuiert ist, inaktiviert.

Die Erfindung betrifft insbesondere auch ein Verfahren zur Inaktivierung von nicht-umhüllten Viren in einem proteinhältigen biologischen Material, welches sich dadurch auszeichnet, daß einem biologischen Material eine photodynamische Substanz, ausgewählt aus der Gruppe der Phenothiazine, in einer Konzentration von mindestens 20 µM zugesetzt wird und die Mischung einem Laserstrahl im Wellenbereich der maximalen Aktivierungsrate der photodynamischen Substanz mit einer Intensität des Laserlichtes von ≤ 0,1 J/cm² pro Zyklus, vorzugsweise ≤ 0,06 J/cm² pro Zyklus für mindestens 20 Zyklen ausgesetzt wird. Bevorzugte, zu inaktivierende Viren sind dabei Viren aus der Gruppe der Picornaviridae und Parvoviridae.

Die Inaktivierung bzw. Desintegration der Nucleinsäure wird vorzugsweise durch ein Verfahren zur Quantifizierung von Nucleinsäuren kontrolliert, wobei ein biologisches Material erhalten wird, dessen Gehalt an Nucleinsäure unterhalb von 100 pg, vorzugsweise von 10 pg, besonders bevorzugt von 1 pg pro Dosis bzw. maximal 1 bis 500 Kopien/ml aufweist und damit im wesentlichen frei von Nucleinsäure-Template-Aktivität ist.

Gemäß einem anderen Aspekt der Erfindung wird das erfindungsgemäße Verfahren zur Herstellung einer inaktivierten Virus-Vakzine vorgesehen.

Das erfindungsgemäße Verfahren ist zur Erzeugung von Vakzinen gegen eine große Vielfalt von Viren, einschließlich menschlicher Viren und tierischer Viren, geeignet.

Daher betrifft die vorliegende Erfindung weiters den Einsatz des erfindungsgemäßen Verfahrens zur Herstellung einer inaktivierten Virus-Vakzine, welches dadurch gekennzeichnet ist, daß einem virushältigen Material eine photodynamische Substanz, ausgewählt aus der Gruppe der Phenothiazine, zugesetzt wird, die Mischung einer Bestrahlung mit Laserlicht mit einer Intensität von ≤ 0,1 J/cm² pro Zyklus ausgesetzt wird, um im wesentlichen die gesamte biologisch aktive Nucleinsäure im biologischen Material zu desintegrieren bzw. zu inaktivieren, während die biologische Integrität und Aktivität, wie Antigenität und Immunogenität des Virus im wesentlichen erhalten bleibt, die Desintegration der Nucleinsäure durch ein Verfahren zur Quantifizifierung von Nucleinsäuren kontrolliert wird und ein virushältiges Material erhalten wird, dessen Gehalt an Nucleinsäure unterhalb von 100 pg, vorzugsweise von 10 pg, besonders bevorzugt von 1 pg pro Dosis bzw. maximal 1 bis 500 Kopien/ml aufweist und damit im wesentlichen frei von Nucleinsäure-Template-Aktivität ist, die inaktivierten Viren gegebenenfalls einer weiteren Reinigung unterzogen werden und die inaktivierten Viren mit einem physiologisch akzeptablen Träger zu einer Virus-Vakzine formuliert werden.

Das Verfahren ist zur Inaktivierung doppelsträngiger DNA-Viren, einzelsträngiger DNA-Viren, doppelsträngiger RNA-Viren und einzelsträngiger RNA-Viren, einschließlich sowohl umhüllter als auch nicht-umhüllter Viren, wie etwa ausgewählt aus der Gruppe Adenovirus, Herpesvirus, Papovavirus, Poxvirus, Parvovirus, Reovirus, Retrovirus, Myxovirus, Paramyxovirus, Picornavirus, Togavirus, Flavivirus, Orthomyxovirus oder Rhadovirus, geeignet. Insbesondere ist das Virus ein Influenza-Virus, Herpes-simplex-Virus, HIV, HBV, HAV, HCV, HSV, TBEV oder CMV, wobei das Virus gegebenenfalls attenuiert ist.

Die vorliegende Erfindung ist auch zur Herstellung von virusvakzineh geeignet, bei denen das zu inaktivierende Virus ein abgeschwächtes Virus ist. Dies ist besonders in einem solchen Fall vorteilhaft, in welchem das ursprünglich aktive Virus hoch virulent ist, z.B. HIV oder HCV, so daß der Herstellungsprozeß an sich Sicherheitsprobleme für das Laborpersonal verursachen könnte, indem dieses mit hochtitrigen Virus-Präparationen arbeitet. Durch Verwendung eines abgeschwächten Virusstammes für den Vakzin-Erzeugungsprozeß ist das Risiko geringer als bei Verwendung des virulenten Stammes. Außerdem würde die Verwendung eines ab geschwächten Virus für die Vakzinherstellung anstelle der virulenten Form die Sicherheit eines solchen Vakzins maximieren, obwohl mittels des vorliegenden Verfahrens ein vollständig inaktiviertes, nicht-infektiöses Virus, bei dem alle biologisch aktiven Nucleinsäuren desintegriert sind, erhalten wird.

Bei der erfindungsgemäßen Herstellung der Vakzine können ausreichende Mengen an zu inaktivierendem Virus beispielsweise erhalten werden, indem Virus aus stark kontaminiertem Plasma konzentriert wird. Stark kontaminiertes Plasma kann auf herkömmliche Weise fraktioniert werden, indem Ethanol/PEG-Fällung, Sephadex-Adsorption und andere herkömmliche Reinigungsschritte verwendet werden, um hoch-konzentrierte Präparationen dieser Viren herzustellen, welche dann durch Behandlung mit einer photodynamischen Substanz und/oder mit Laserstrahl inaktiviert werden können. Viren können auch durch Verwendung von Nanofiltrationstechniken konzentriert werden, wobei gegebenenfalls kleine Viren, wie Parvovirus, mit spezifischen Antikörpern komplexiert werden können, um einen Immunkomplex zu schaffen, welcher durch Nanofiltration zurückgehalten werden kann. Diese Technik wird vorzugsweise für Viren verwendet, welche in Zellkulturen nicht gezüchtet werden können, wie HCV, HBV oder Parvovirus. Die vorliegenden Vakzine können auch durch Züchtung von Impfvirus in einer geeigneten Vertebraten-Zellkultur erhalten werden. Impfvirus kann durch direkte Isolation aus einem infizierten Wirt oder aus einer Quelle, wie einer Sammlung von Mikroorganismen erhalten werden. Zu den geeigneten Zellkulturen zählen primäre oder sekundäre Kulturen, die von Geweben oder etablierten Zellinien stammen, wie CEC, Vero-Zellen, Affennierenzellen, BHK-Zellen, CV-1-Zellen, LLC-MK-2-Zellen, MDCK-Zellen, MDBK-Zellen, LTMK-Zellen, WI-38-Zellen, MRC-5-Zellen oder anderen Zellen, die für das gewünschte Virus permissiv sind und die als Einzelschichten oder Suspensionskultur gezüchtet werden können. Die Zellkultur wird entweder mittels herkömmlicher Verfahren, wie dem Züchten von Zellen in serumhältigem Medium erhalten, oder insbesondere werden sie unter serum-oder proteinfreien Bedingungen aus der Originalampulle zu einer Zell-Biomasse in großem Umfang gezüchtet.

Daher wird die virusinfektion vorzugsweise unter serum- oder proteinfreien Bedingungen durchgeführt. Dies kann durch Züchten der Zellkultur in serum- oder proteinfreiem Medium für mehrere Generationen vor der Infektion, oder durch Ersetzen von serumhältigem Wachstumsmedium durch serum- oder proteinfreies Medium vor der Infektion erfolgen. Zellen werden unter Standardbedingungen mit einer Vielzahl von Infektionen (multiplicity of infection, m.o.i.), vorzugsweise zwischen 0,001 und 0,1 TCID₅₀, insbesondere etwa 0,01, mit dem Virus infiziert. Zellwachstum, Infektion und Viruserzeugung werden periodisch überwacht. Die Überwachung kann automatisch oder mit anderen Mitteln erfolgen, und die Ergebnisse der Überwachung können zur Steuerung des Herstellungsprozesses verwendet werden. Durch Infektion und Inkubation der Zell-Biomasse mit dem Virus wird eine Zellkultur, die einen virushältigen Überstand aufweist, und/oder eine Zellbiomasse, die Virus und Virus-Antigen aufweist, erhalten. Je nach der Art des verwendeten Virus findet man die erzeugten Virus-Teilchen entweder im Überstand der Zellkultur und/oder in Verbindung mit der Zell-Biomasse.

Beispiele für lytische Viren schließen Influenza-Virus und Vaccinia-virus ein. Das Virus wird erzeugt und in das Zellkulturmedium abgegeben, um ein virushältiges Medium zu erzeugen. Das virushältige Medium kann von der Zellkultur abgetrennt und entweder direkt dem Laserstrahl ausgesetzt werden, oder gegebenenfalls vor der Laserstrahl-Einwirkung mit einer gewählten Konzentration einer photodynamischen Substanz behandelt werden. Das virushältige biologische Material kann portionsweise oder vollständig in einem kontinuierlichen Verfahren oder chargenweise aus dem Zellkulturgefäß entfernt und in einen zweiten Behälter transferiert werden, welchem die photoaktive Substanz zugesetzt wird. Die photodynamische Substanz kann der Virussuspension in einer einzigen Zugabe oder in mehreren Zugaben zugesetzt werden, wobei das Virus zwischen den Zugaben einem Laserstrahl ausgesetzt wird, oder sie kann kontinuierlich während der gesamten Behandlungsdauer oder einem Teil derselben zugesetzt werden. Üblicherweise ist die Anzahl der Zugaben 1. Gemäß der vorliegen den Erfindung kann dies durch Rezyklieren der photodynamischen Substanz und des Laserstrahl-behandelten Mediums in einem kontinuierlichen Prozeß erfolgen. Das virushältige Medium wird aus dem Kulturgefäß in einen Behälter transferiert, in welchem die photodynamische Substanz in einer gewählten Konzentration zugegeben wird, und diese Mischung wird durch ein System mit einem Fenster zwecks Laserstrahl-Einwirkung während einer Zeit, die ausreicht, um im wesentlichen die gesamte biologisch aktive Nucleinsäure zu zersetzen, gepumpt.

Gemäß dem Verfahren der Erfindung können während aller Stufen des Virusinaktivierungsverfahrens ein oder mehrere Quencher und/oder Scavenger und/oder Stabilisatoren alleine oder in jeder geeigneten Kombination zugegeben werden. Das inaktivierte Virus wird weiters einer Reinigung unterzogen, oder das virushältige Material wird direkt zum zweiten Behälter zurückgepumpt oder für einen zweiten Laserstrahl-Einwirkungszyklus in umgekehrter Richtung durch das Fenster gepumpt, wobei eine zweite Portion von photodynamischer Substanz und/oder Quencher und/oder Scavenger und/oder Stabilisator zugegeben oder auch nicht zugegeben werden kann, und einem zweiten Laserstrahl-Zyklus unterzogen. Dieses Verfahren kann für mehrere Zyklen, vorzugsweise 5 bis 50 Zyklen, insbesondere 10 bis 30 Zyklen, wiederholt werden.

Während der Laserstrahl-Bestrahlung kann das Medium ruhig, gerührt oder im Kreislauf gehalten werden, und es kann entweder einer kontinuierlichen Einwirkung oder abwechselnden Perioden von Einwirkung und Nichteinwirkung unterzogen werden. Der Kreislauf kann in einem System mit geschlossenem Wirkungskreis oder in einem Einzelsystem erfolgen, wobei gewährleistet ist, daß alle Proben dem Licht ausgesetzt worden sind.

Während der Infektion und der Vermehrung werden nicht immer alle von der Zelle produzierten Virusteilchen in den Überstand freigesetzt. Diese Virusteilchen können in den Zellen der Zell-Biomasse verbleiben. Daher enthält die Zellkultur Virusteilchen im Überstand und vollständige Virusteilchen sowie Virusproteine in Verbindung mit der Zell-Biomasse. Um eine größere Virusausbeute zu erhalten, werden die Virusteilchen aus dem Überstand geerntet, und das Virus und/oder Virus-Antigen, das mit der Zell-Biomasse verbunden ist, wird isoliert. Das in den Zellen der Zell-Biomasse gefundene Virus wird durch Lyse aus den Zellen freigesetzt. Die Zellen können mittels herkömmlicher Methoden, wie Behandlung der Zellen mit einem Detergens, Behandlung mit Hitze, Ultraschallbehandlung, French-Press oder anderen Zell-Lysemethoden lysiert werden. Die aus den Zellen freigesetzten Viren werden beispielsweise mittels Zentrifugation geerntet und in einem Puffer resuspendiert. Virale Antigene, die noch mit der Zell-Biomasse oder mit Zellfragmenten verbunden sind, können mittels zum Stand der Technik gehörender chemischer oder mechanischer Methoden aus den Zellen oder Zellfragmenten extrahiert werden. Zu diesen Methoden zählen die Ultraschallbehandlung oder die Behandlung mit einem geeigneten Detergens, um das Virus-Antigen aus der Zelle oder den Zellfragmenten, insbesondere von der Membran, freizusetzen. Die Nucleinsäuren in dieser Suspension, die entweder von den Zellen oder vom Virus stammen, werden durch Laserstrahl-Einwirkung oder durch Behandlung der Lösung mit einer vorgewählten Konzentration einer photodynamischen Substanz und nachfolgende Laserstrahl-Einwirkung desintegriert. Das Virus-Antigen, einschließlich Viren, die aus der Zell-Biomasse isoliert sind, können dann weiters einem Reinigungsschritt ein schließlich Auftrennung auf einem Saccharose-Gradienten, Adsorption an einer Chromatographiesäule, Waschen und Elution des gereinigten Virus oder Virus-Antigens unterzogen werden. Die verwendete Chromatographiesäule ist ausgewählt aus Ionenaustausch-Chromatographie, Affinitäts-Chromatographie oder Größenfiltrations-Chromatographie.

Das virushältige Medium kann auch durch Zentrifugation und Suspension in einem wässerigen gepufferten Medium durch einen anorganischen Puffer ersetzt werden. Der anorganische Puffer ist vorzugsweise ein Phosphat-Puffer oder Tris/HCl. Die Menge der Virusausbeute in der Suspension ist im allgemeinen etwa 10⁶ bis 10⁹ pfu/ml, vorzugsweise etwa 10⁷ bis 5 x 10⁸ pfu/ml.

Weiters umfaßt die Erfindung den Schritt der Herstellung eines Vakzins mit dem inaktivierten Virus/Virus-Antigen.

Obwohl die Desintegration von Nucleinsäure in einem Mikroorganismen-enthaltenden biologischen Material gemäß der vorliegenden Erfindung normalerweise in einer Inaktivierungs- oder Desintegrationslösung durchgeführt wird, kann es in einigen Fällen erwünscht sein, die photodynamische Substanz direkt in das zellkulturmedium, in welchem das virus gezüchtet wird, einzubringen. Unter solchen Bedingungen kann die Zellkultur über mehrere Generationen in Serum- oder Protein-freiem Medium in Anwesenheit einer photodynamischen Substanz, vorzugsweise vor der Infektion, im Dunklen und unter optimalen Wachstumsbedingungen gezüchtet werden. Nach der Virusinfektion und der Virusvermehrung erfolgt die Nucleinsäure-Desintegration durch Entfernen des lebendes Virus enthaltenden Mediums, das mit photodynamischer Substanz aus der Zellkultur behandelt worden ist oder auch nicht, und Einwirkung eines Laserstrahls einer gewählten Wellenlänge auf die viruspartikel im Inaktivierungsmedium, in welchem eine zusätzliche photodynamische Substanz und/oder ein Quencher und/oder ein Scavenger und/oder ein Stabilisator enthalten oder auch nicht enthalten sein können.

Mit dem Verfahren der vorliegenden Erfindung ist die Möglichkeit, weniger rigorose Inaktivierungsbedingungen verwenden zu können, von großem Vorteil bei der Erhaltung der Antigenität, Immunogenität und Protektivität des Virus während der Desintegration der viralen Nucleinsäure. Überraschenderweise zeigte es sich, daß unter den vorliegenden Bedingungen, wenn Virus mit einer photodynamischen Substanz behandelt und mit Laserstrahl bestrahlt wird, bestimmte Viren, insbesondere Non-Lipidviren mit starren Kapsiden inaktiviert werden können, wobei sie ihre Infektiosität vollständig verlieren. In einigen Fällen kann es erwünscht sein, ein organisches Lösungsmittel zuzugeben, um die Permeabilität der äußeren Hülle oder Membran des Virus zu erhöhen. Eine solche Erhöhung der Permeabilität erleichtert das Eindringen der photodynamischen Substanz und verbessert die Desintegration der viralen Nucleinsäure im inneren Kern des Virus. Die photodynamische Substanz und das organische Lösungsmittel können auch zum Zellkulturmedium, in dem das Virus gezüchtet wird, zugegeben werden.

Es mag erwünscht sein, die verbrauchte photodynamische Substanz und/oder ihre Photozerfallsprodukte aus der Mischung zu entfernen. Dies kann durch ein oder mehrere Standard-Laborverfahren, wie Dialyse über einer entsprechend großen Membran oder einem Hohlfasersystem nach Beendigung der Laserstrahl-Einwirkung erfolgen. Alternativ könnte man Affinitäts- oder Gelfiltrations-Chromatographiemethoden für ein oder mehrere zu entfernende Materialien mit niedrigem Molekulargewicht verwenden.

Erfindungsgemäß können Viren, die gegen eine Inaktivierung und Desintegration der Nucleinsäure resistent waren oder nur ineffizient inaktiviert werden konnten, ohne Verlust der gewünschten biologischen Aktivität vollständig inaktiviert werden. Viren, die bisher erfolgreich inaktiviert wurden, können nun unter Bedingungen inaktiviert werden, bei welchen ihre antigenen Charakteristika besser erhalten bleiben, wodurch sie zu effizienteren immunogenen Substanzen zur Verwendung in Vakzinen werden.

Das inaktivierte Virus kann auf vielfältige Weise zur Verwendung als Vakzin formuliert werden. Die Konzentration des Virus ist im allgemeinen von 10⁶ bis 10⁹ pfu/ml bei einer Gesamtdosierung von mindestens 10⁵ pfu/ml, üblicherweise mindestens 10⁶ pfu/ml, vor zugsweise mindestens 10⁷ pfu/ml. Geeignete Vakzine können durch Kombination der inaktivierten Viren mit einem physiologisch akzeptablen Träger, typischerweise einem Adjuvans, in einer geeigneten Menge zur Bewirkung einer Immunreaktion hergestellt werden, z.B. der Produktion von Serum-neutralisierenden Antikörpern, nach nachfolgender Impfung des Wirtes. Es kann in einem inerten, physiologisch akzeptablen Medium, wie ionisiertem Wasser, Phosphatgepufferter Kochsalzlösung, oder Kochsalzlösung vorliegen, oder kann in Kombination mit einem physiologisch akzeptablen immunologischen Adjuvans, einschließlich jedoch nicht beschränkt auf Mineralöl, Freund'sches Adjuvans, Pflanzenöle, Mineralsalze und Immunpotentiatoren, verabreicht werden. Das Vakzin kann subkutan, intramuskulär, intraperitoneal, oral oder nasal verabreicht werden. Gewöhnlich liegt eine spezifische Dosierung an der spezifischen Stelle im Bereich von etwa 0,1 bis 1,0 ml, wobei die Gesamtdosierung im Bereich von 1,0 ml bis 10 ml liegt. Die Anzahl der Injektionen und ihr zeitlicher Abstand kann stark variieren, doch üblicherweise sind 1 bis 3 Injektionen in 1-, 4- und 24-Wochen-Intervallen wirksam.

Das erfindungsgemäß hergestellte Vakzin kann zur Behandlung einer Virusinfektion und/oder zur Prophylaxe einer Virusinfektion durch Verabreichung eines wie oben beschrieben hergestellten Vakzins an ein Tier oder einen Menschen verwendet werden.

Die Verringerung oder der Verlust der Infektiosität und die vollständige Inaktivierung von Viren wird auf herkömmliche Weise durch Blind-Passage von Proben bestimmt, wobei ein biologischer Test, wie ein Enzymtest (z.B. Reduktion in reverser Transcriptase) oder die Bestimmung vor Virus-Titer, CPE, TCID₅₀, pfu/ml oder in einem Tiermodell verwendet wird. Eine empfindlichere Methode ist von Hanson et al. (1990, J. Clin. Microbiol. 28: 2030-2034) beschrieben. Tatsächlich stellte sich heraus, daß bei dieser Vorgangsweise der Messung der Virusinaktivierung typischerweise nicht unterschieden werden kann zwischen einer Situation, in welcher die Inaktivierung des Virus vollständig ist, und jener Situation, in welcher die Inaktivierung nicht vollständig ist und die restliche biologisch aktive virale Nucleinsäure noch in vivo infektiös sein könnte. Zur Herstellung eines inaktivierten Virus-Vakzins von beispielsweise HIV wäre es sehr erwünscht, zu gewährleisten, daß die Infektiosität verloren ist, und daß die gesamte aktive Nucleinsäure des Virus vollständig desintegriert ist. Dies erfordert die Verwendung eines verbesserten Inaktivierungsverfahrens und die Quantifizierung der restlichen biologisch aktiven Nucleinsäure mit einem hochempfindlichen, genauen und reproduzierbaren Verfahren.

Bachmann et al. (1995, J. Med. Virol. 47:172-178) offenbarten die Verwendung des PCR-Inhibitionstests zur Bestimmung der Menge der viralen RNA nach Inaktivierung mit Methylenblau und Licht. Sie konnten nach 25 Amplifikationszyklen keine virale Nucleinsäure entdecken, wogegen spezifische Amplifikationsprodukte gefunden wurden, wenn die RNA 30 Zyklen lang amplifiziert wurde. Daher ermöglichte der Nucleinsäure-Quantifizierungstest keine genaue Bestimmung des Inaktivierungsverfahrens und lieferte auch keinen Beweis dafür, daß die Inaktivierung der Nucleinsäure mittels dieses Verfahrens vollständig war. Man braucht daher ein umfassendes und quantitatives Verfahren zur Bestimmung der Inaktivierungseffizienz.

Die vorliegende Erfindung umfaßt das Messen der Inhibition der Template-abhängigen enzymatischen Nucleinsäure-Synthese nach Laserstrahl-Einwirkung auf ein biologisch aktives Material, gegebenenfalls nach Zugabe einer photoaktiven Substanz, die an Nucleinsäure bindet, und nach Laserstrahl-Einwirkung, um die aktive Nucleinsäure zu desintegrieren. So würden also, wenn die Nucleinsäure mittels des Verfahrens der vorliegenden Erfindung desintegriert wird, Versuche zur Amplifizierung der Nucleinsäure zu keinem Amplifikationsprodukt führen. Die Reagenzien für die Amplifikation von beispielsweise viraler Nucleinsäure sind typischerweise spezifisch für das Virus und/oder die Zell-Nucleinsäure der Zellkultur, die entweder für die Virusvermehrung oder für die Erzeugung eines gentechnisch hergestellten Produkts verwendet wird.

Die Effizienz des Desintegrationsprozesses kann dadurch bestimmt werden, daß es nicht möglich ist, amplifizierte Produkte der Polymerase-Kettenreaktion nachzuweisen. Die Wirksamkeit des PCR-Verfahrens kann durch Verwendung mindestens zweier verschiedener interner Standards, die sich von der zu amplifizierenden Sequenz durch mindestens einen Marker unterscheiden, kontrolliert werden.

Die Desintegration biologisch aktiver Virus-Nucleinsäure in der Desintegrationsmischung wird durch die Schritte des Vorsehen eines virushältigen biologischen Materials, das zur Desintegration biologisch aktiver Nucleinsäure behandelt worden ist, des Behandelns einer Probe der Mischung, so daß die Virus-Nucleinsäure aus den Viruspartikeln freigesetzt wird, der Zubereitung einer Suspension, so daß die Nucleinsäure des Virus amplifizierbar ist, und der Zugabe eines Satzes von Primern und mindestens eines inneren Standards, der sich von der zu amplifizierenden Nucleinsäure in mindestens einem Marker unterscheidet, zur Mischung, und der Durchführung einer Amplifikationsreaktion, die ausreicht, um Amplifikationsprodukte auszubilden, bestimmt. Die Nucleinsäure, die dabei bestimmt und in einer aus einem Zellkultursystem stammenden Virussuspension mit desintegrierter Nucleinsäure amplifiziert werden soll, ist die vom Virus stammende Nucleinsäure und die aus der Zellkultur stammende Nucleinsäure.

Die vorliegende Erfindung sieht ein Verfahren zur Herstellung einer sicheren, inaktivierten, nicht-infektiösen Vakzine vor, mit welchem die Risken der Übertragung von biologisch aktiver Nucleinsäure auf den geeimpften Wirt eliminiert werden. Die vorliegende Erfindung sieht auch ein Verfahren zur Erzeugung von sicherem, nicht-infektiösem biologischen Material vor.

Gemäß der vorliegenden Erfindung wird der Nucleinsäuregehalt an aktiver Nucleinsäure in einem biologischen Material bestimmt und nach dem Desintegrationsschritt mittels einer Nucleinsäure-Amplifikationsmethode, vorzugsweise PCR, quantifiziert. Der Parameter der Desintegrationskapazität ist der vollständige Verlust des Nachweises von aktiver Nucleinsäure, festgestellt mittels PCR.

Durch diese Vorgangsweise kann die Desintegration der Nucleinsäure kontrolliert werden. Vorzugsweise erfolgt die Durchführung des Verfahrens derart, daß nach der Behandlung mit photoaktivem Agens und Laserlicht der Gehalt an nachzuweisender Nucleinsäure unterhalb von 100 pg, vorzugsweise von 10 pg, besonders bevor zugt von 1 pg pro Dosis bzw. unterhalb von 500 Kopien/ml, vorzugsweise von 300 Kopien/ml bzw. maximal 1 bis 500 Kopien/ml liegt. Der Nucleinsäuregehalt liegt vorzugsweise unterhalb der Nachweisgrenze eines gemäß dem Stand der Technik sehr sensitiven Nucleinsäure-Quantifizierungsverfahren.

Wenn nach der Desintegration noch eine Nucleinsäure-Template-Aktivität mittels PCR gemessen wird, wird das Desintegrationsverfahren an Bedingungen angepaßt, die die vollständige Reduktion der Nucleinsäure-Template-Aktivität ermöglichen.

Wenn das vorliegende Verfahren der Desintegration biologisch aktiver Nucleinsäure verwendet wird, um z.B. Pathogene zu inaktivieren, die entweder für die Herstellung von Vakzinen oder zum Erhalt von nicht-infektiösem biologischem Material verwendet werden, ist es wesentlich, daß die Desintegration der biologisch aktiven Nucleinsäure vollständig ist.

Das Verfahren wird daher als Qualitätskontrolltest verwendet, um die angemessene Behandlung eines biologischen Materials mit Laserstrahl oder photodynamischer Substanz/Laserstrahl zu verifizieren.

Wenn das Verfahren zur Herstellung eines Vakzins verwendet wird, wird die Immunogenität nach Laserstrahl- oder photodynamischer Substanz/Laserstrahl-Desintegration gewöhnlich durch Immunisierungs- und Schutzuntersuchungen an Labortieren, wie Mäusen oder Kaninchen, Bestimmung des bindenden Antikörpertiters und Neutralisierungstests beurteilt. Um zu gewährleisten, daß das inaktivierungsverfahren die Integrität und Aktivität, wie die antigenen und immunogenen Eigenschaften des biologischen Mittels nicht verändert hat, wurde auch ein empfindlicheres Verfahren zur Bestimmung der Antigenität verwendet. Die Erhaltung der biologischen Integrität und Aktivität, wie Antigenität und Immunogenität von gemäß dem Verfahren der Erfindung behandelten biologischen Produkten wird durch Epitop-Kartierung und Wirksamkeitstest oder durch einen Aktivitätstest getestet.

Die Erfindung wird anhand der folgenden Beispiele und der Zeichnungsfigur näher beschrieben, auf welche sie jedoch nicht eingeschränkt werden soll.

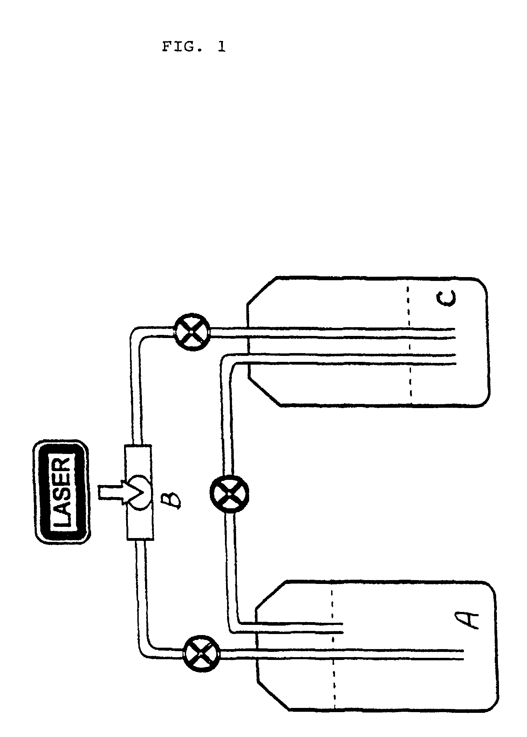

Es zeigt Fig. 1: Ein System zur Laserstrahl-Aktivierung einer photodynamischen Substanz, die einem biologischen Material zugegeben worden ist.

Eine photodynamische Substanz wird einem Behälter (A) zugegeben, welcher das biologische Material enthält. Dieses wird durch ein System mit einem Fenster (B) zwecks Einwirkung eines Laserstrahls in einen zweiten Behälter (C) gepumpt. Es kann in umgekehrter Richtung zurückgepumpt werden oder rezirkuliert werden, um zu ermöglichen, daß die Probe mehreren Zyklen einer Laserstrahl-Behandlung unterzogen wird.

### Beispiele:

### Beispiel 1: Inaktivierung von HIV-1, TBEV, Influenzavirus, HSV-1 und Poliovirus durch Methylenblau/ Laserstrahlbehandlung (nach Ansicht der Anmelderin derzeit der beste Weg zur Ausführung der Erfindung)

Eine schematische Beschreibung dieser Vorgangsweise ist in Fig. 1 gezeigt. Dabei wird ein System eingesetzt, bei dem das biologische Material über ein Röhrchen oder eine Küvette mit einem Glasfenster geleitet wird und das Glasfenster mit einem Laserstrahl bestrahlt wird. Die Energiedichte, die auf das biologische Material einwirkt, wird durch die eingesetzte Strahlungsintensität des Lasers x Verweildauer der Flüssigkeit im Laserstrahlungsbereich bestimmt. Die Strahlungsintensität des Lasers ist dabei die Laserleistung pro Laserstrahlungsfläche. Die Verweildauer im Strahl ist dabei abhängig von der Geometrie des Glasfensters und der Durchflußrate.

100 ml Virus-Stammlösung werden in einem Kolben gelassen, der unter Rühren auf 4°C gehalten wird. Eine Methylenblau-Stammlösung mit einer Konzentration von 10 mM wird auf eine Endkonzentration von 2 µM zugegeben. Nach gründlichem Rühren wird das Material durch ein Röhrchen aus rostfreiem Stahl mit einem Glasfenster, durch welches es mit einem Laserstrahl von einem He-Ne-Laser mit einer Wellenlänge von 633 nm und mit einer Arbeitsintensität von 10 mW bestrahlt wird, gepumpt. Bei einem quadratischen Durchmesser des Glasfensters von 2 x 2 mm und einer Durchflußrate von 4 ml/min ergibt sich eine Fließgeschwindigkeit durch den Strahlungsbereich und damit eine Verweildauer von 0,06 sec/mm. Ausgehend von einer Laserleistung von 10 mW ist die Laser-Intensität pro Zyklus damit 0,6 mWs/mm² oder 0,06 J/cm². Das Material wurde 0 bis 10 Bestrahlungszyklen lang rezirkuliert, und Proben wurden nach der Anzahl der Zyklen zur Virustitrierung entnommen, wie oben beschrieben. Kontrollen mit Methylenblau wurden 30 Zyklen unter identischen Bedingungen, jedoch ohne Bestrahlung unterzogen. Die Virus-Titer wurden vor und nach Methylenblau/Laser-Behandlung bestimmt und sind in Tabelle 1 zusammengefaßt.

**Tabelle 1:**

| **Inaktivierung von HIV-1, TBEV, Influenza-, HSV-1 und Polio-Virus durch Methylenblau/Laserstrahl - Behandlung Light Treatment** | | | | | |
|---|---|---|---|---|---|
| **Behandlung** | **HIV-1** | **HSV-1** | **TBEV** | **Influenza** | **Polio** |
| **keine** | 10^{7.0} | 10^{8.4} | 10^{7.3} | 10^{8.2} | 10^{8.9} |
| **1 Zyklus** | 10^{6.5} | 10^{7.5} | 10^{6.2} | 10^{7.7} | 10^{8.7} |
| **2 Zyklen** | 10^{6.1} | 10^{6.0} | 10^{5.7} | 10^{5.5} | 10^{8.6} |
| **5 Zyklen** | 10^{1.9} | 10^{2.0} | 10^{2.9} | 10^{1.7} | 10^{8.9} |
| **10 Zyklen** | <10^{0.2} | <10^{0.2} | <10° | <10^{0.2} | 10^{8.8} |
| **20 Zyklen** | <10^{0.2} | <10^{0.2} | <10° | <10^{0.2} | 10^{6.4} |
| **30 Zyklen** | <10^{0.2} | <10^{0.2} | <10⁰ | <10^{0.2} | 10^{4.4} |
| **30 Zyklen mit Laserstrahl/ohne MB** | 10^{6.4} | 10^{7.9} | 10^{7.0} | 10^{7.1} | 10^{8.2} |
| **30 Zyklen ohne Laserstrahl und MB** | 10^{6.9} | 10^{8.1} | 10^{7 1} | 10^{7.6} | 10^{8.1} |

### Beispiel 2: Inaktivierung von Polio-Virus mit Methylenblau/Laserstrahl-Behandlung mit erhöhter Konzentration von Methylenblau

Eine Methylenblau-Stammlösung wurde zu 100 ml einer Polio-Virus-Stammlösung auf Endkonzentrationen von 1, 3, 10 bzw. 30 µM zugegeben. Jede Probe wurde, wie in Beispiel 1 beschrieben, 30 Zyklen lang jeweils mit 0,06 J/cm² bestrahlt, Die Virus-Titer wurden nach Bestrahlung bei jeder Methylenblau-Konzentration bestimmt. Es konnte gezeigt werden, daß nicht-umhülltes Polio-Virus mit mehr als 8 log-Stufen inaktiviert wird.

**Tabelle 2:**

| **Inaktivierung von Polio-Virus mit Methylenblau/ Laserstrahl-Behandlung** | |
|---|---|
| MB-Konz. (µM) | Virus-Titer |
| 1 | 10^{8,9} |
| 3 | 10^{6,2} |
| 10 | 10^{2,7} |
| 30 | <10^{0,2} |

### Beispiel 3 : Vergleich der Antigenität von mit Methylenblau/Laserstrahl und mit Formalin inaktivierten Vakzin-Präparationen

Saccharose-Gradient-gereinigte Ganzvirus-Präparationen von HIV-1, Influenza A/FPV/Rostock/34, einem TBE-Virus und einer gereinigten Virus-Glycoprotein-Untereinheit-Präparation aus mit HSV-1 infizierten Vero-Zellen wurden 30 Zyklen einer Methylenblau (2µM)/Laserstrahl (10mW)-Behandlung unterzogen. Identische Präparationen wurden durch Formalinbehandlung (0,05% Endkonzentration, 30 h bei 37°C) inaktiviert. Nach einem Sicherheitstest, wie in Beispiel 1 beschrieben, um zu gewährleisten, daß kein infektiöses Virus vorhanden war, wurde die Antigenität aller Präparationen bestimmt.

Die Antigenität der HIV-1-Präparationen wurde durch Verwendung des handelsüblichen Antigen-Capture-ELISA bestimmt. Die mit Methylenblau/Laserstrahl inaktivierten Präparationen wurden seriell zweifach verdünnt, bevor 100 µl jeder Verdünnung in eine zuvor mit gereinigtem anti-HIV-1-IgG vorbeschichtete Vertiefung gegeben wurden. Die Platten wurden dann gründlich gewaschen, und 100 µl Human-anti-HIV-1-Antikörper, der kovalent an Peroxidase gebunden war, wurden zugegeben und bei Raumtemperatur 90 min lang inkubiert. 200 µl aktiviertes Substrat (O-Phenylendiamin) wurden dann zugegeben und inkubiert, bis die Reaktion durch Zugabe von 50 µl 8M Schwefelsäure gestoppt und die Extinktion bei 490 nm abgelesen wurde. Der Antigentiter wurde als der reziproke Wert der höchsten Verdünnung, die eine positive Reaktion ergab, bestimmt.

Die TBEV-Antigenität wurde auch durch Antigen-Capture-ELISA unter Verwendung eines gereinigten Meerschweinchen-anti-TBEV-IgG als Capture-Antikörper bestimmt. Die inaktivierten Präparationen wurden seriell zweifach verdünnt, und 100 µl jeder Verdünnung wurden zugegeben und 90 min lang bei 37°C inkubiert. Nach dem Waschen wurden 100 µl Kaninchen-anti-TBEV-Serum zugegeben, und nach 60minütiger Inkubation wurden 100 µl Peroxidase-gebundenes Anti-Kaninchen-IgG zugegeben. 200 µl aktiviertes Substrat (O-Phenylendiamin-Hydrochlorid) wurden dann zugegeben, und es wurde im Dunklen inkubiert, bis die Reaktion durch Zugabe von 50 µl 8M Schwefelsäure gestoppt wurde und die Extinktion bei 490 nm abgelesen wurde. Zusätzlich wurde eine hoch-reine TBEV-Antigen- Präparation im ELISA als Standard verwendet, was eine direkte Berechnung der Menge des reaktiven TBEV-Antigens ermöglichte.

Die HSV-Antigen-Reaktivität wurde ebenfalls gemessen, wobei ein modifiziertes handelsübliches ELISA-Set verwendet wurde. Die Antigenproben wurden seriell zweifach verdünnt, und 100 µl jeder Verdünnung wurden auf mit gereinigtem Kaninchen-anti-HSV-1-IgG vorbeschichtete Platten gegeben. Nach Inkubation und Waschen wurde ein mit Peroxidase-konjugierter anti-HSV-1-Antikörper zugegeben. Dieser wurde bei 37°C 1 h lang inkubiert, und nach dem Waschen wurden 100 µl aktiviertes Substrat (O-Phenylendiaminhydrochlorid) zugegeben und im Dunklen 10 min lang inkubiert. Die Reaktion wurde durch Zugabe von 50 µl 5M Schwefelsäure gestoppt, und die Extinktion wurde bei 490 nm abgelesen. Der Antigentiter wurde nach der Erstellung einer Standardkurve unter Verwendung von hochreinem HSV-1-Antigen-Standard, dessen Reaktivität im ELISA-System bestimmt wurde, bestimmt. Die Antigenität von Influenza wurde mittels des Hämagglutinationstests, d.h. Agglutination von Virus mit Erythrozyten, gemessen. Dies wurde wie von Hirst "The Agglutination of Red Cells by Allantoic Fluid of Chicken Embryos Infected with Influenza Virus", Science 94: 22-23 (1941) beschrieben, durchgeführt. Die Ergebnisse sind in Tabelle 3 zusammengefaßt.

**Tabelle 3:**

| **Vergleich der Antigenität von mit Methylenblau/Laserstrahl und Formalin inaktivierten Vakzinen** | | | |
|---|---|---|---|
| | Formalin | Methylenblau | |
| HIV | 1:2160 | 1:5120 | Antigen-ELISA-Titer |
| Influenza | 10 | 11 | HA-Titer HAU |
| TBEV | 40,7µg/ml | 39,4µg/ml | Antigen-Konz. |
| HSV | 9001 | 12.370 | ELISA-Einheiten/ml |

### Beispiel 4 : Immunisierung von Mäusen mit Formalin inaktiviertem und mit Methylenblau/Laserstrahl inaktiviertem HIV-1

Eine mittels Saccharose-Gradienten gereinigte HIV-1-Präparation mit einem Titer von etwa 10⁷ TCID₅₀/ml wurde mit einer Formalin-Endkonzentration von 0,05% 30 h lang bei 37°C inaktiviert. Nach dieser Behandlung wurde das Formalin durch Dialyse entfernt. Dieselbe HIV-1-Präparation wurde 10, 20 und 30 Zyklen einer Laserstrahl-Behandlung, wie zuvor beschrieben, nach Zugabe von Methylenblau zum Erhalt einer Endkonzentration von 2µM unterzogen. Das Methylenblau wurde dann durch Leiten über eine G-25 Sephadex-Säule entfernt. Der Proteingehalt aller Präparationen wurde bestimmt und eingestellt. Gruppen von zehn Mäusen wurden mit 10 µg jeder Präparation, mit Al(OH)₃ als Adjuvans, immunisiert, wobei die Mäuse vier Wochen später einen Booster mit derselben Antigen-Dosis erhielten. Die Mäuse wurden zwei Wochen nach der zweiten Immunisierung zwecks Serumpräparation getötet, und nach dem Poolen der Seren der Tiere jeder einzelnen Gruppe wurden die HIV-1-spezifischen Antikörpertiter unter Verwendung des handelsüblichen Gesamtvirus-ELISA-Sets bestimmt. Kurz gesagt wurden die Sera seriell zweifach verdünnt, und 100 µl jeder Verdünnung wurden auf die vorbeschichteten Platten gegeben und 1 h lang bei 37°C inkubiert. Nach gründlichem Waschen wurde jede Vertiefung mit 100 µl Peroxidase konjugiertem Anti-Maus-IgG gefüllt und 1 h lang bei 37°C inkubiert. Nach dem Waschen wurden 200 µl aktiviertes Substrat (O-Phenylendiamin-Hydrochlorid) in jede Vertiefung zugegeben und bei Raumtemperatur im Dunklen 10 min lang inkubiert. Die Reaktion wurde durch Zugabe von 50 µl 5M Schwefelsäure gestoppt, und die Extinktion wurde bei 490 nm abgelesen. Der Antikörpertiter wurde als der reziproke Wert der höchsten Verdünnung, die eine positive Reaktion ergab, bestimmt. Die Ergebnisse sind in Tabelle 4 zusammengefaßt.

**Tabelle 4:**

| **Vergleich der Immunogenität von mit Laserstrahl/** Methylenblau und mit Formalin inaktiviertem HIV-1 | |
|---|---|
| Inaktivierungsmethode | Mittlerer HIV-1-ELISA-Titer |
| 10 Zyklen Laser/MB | 38.000 |
| 20 Zyklen Laser/MB | 40.000 |
| 30 Zyklen Laser/MB | 36.000 |
| Formalin | 36.000 |

### Beispiel 5 : Vergleich der Desintegration von HIV-1-Nucleinsäure durch Methylenblau/Laserstrahl- und durch Formalin-Behandlung

Eine HIV-1-Virus-Präparation wurde durch 30stündige Behandlung mit 0,05% Formalin-Endkonzentration bei 37°C oder durch Behandlung mit Laserstrahl, wie in Beispiel 4 beschrieben, während 10, 20 oder 30 Zyklen, nach Zugabe von Methylenblau zum Erhalt einer Endkonzentration von 2µM inaktiviert. Der Virustiter wurde unter Verwendung einer Standard TCID₅₀-Methodik (Reed et al., 1938, Amer. J. Hyg. 27:493-497) berechnet. Die RNA-Kopienzahl wurde gemäß der folgenden Vorgangsweise bestimmt:

500 µl der inaktivierten Virus-Präparation wurden 20 min lang bei 70.000 U/min in einer Ultrazentrifuge zentrifugiert. Das Pellet wurde in 1 ml Guanidin-Isothiocyanat-Lösung (RNAzol® von Biotec) aufgenommen, und 5 µl von 1mg/ml t-RNA aus Hefe und eine bestimmte Menge, beispielsweise 20 µl, Standard-RNA wurde zugegeben. Eine vorbestimmte Anzahl, z.B. 400 und 1200 Kopien der minus- und plus-RNA-Standards wurden zugegeben und am Vortex geschüttelt. Die Lösung wurde 10 min lang auf 70°C erhitzt, dann wurde 1/10 Volumen Chloroform zugegeben und 10 min lang auf Eis inkubiert. Danach wurde sie in einer Tisch-Zentrifuge 5 min lang zentrifugiert, und der Überstand wurde in neue Gefäße transferiert. 500 µl Isopropanol wurden zugegeben und 15 min lang auf -80°C eingestellt. Danach wurde 10 min lang zentrifugiert, zweimal mit 70% Ethanol gewaschen, und das Pellet wurde in 50 µl Wasser aufgenommen. 5 µl wurden für die RT-PCR verwendet.

Die verwendeten Standard-Plasmide pgag-15 und pgag+12 stammten vom Plasmid pgagl, welches aus dem bekannten pBS/SK-Plasmid (von Stratagene) und einem Insert in der multiplen Klonierungsstelle dieses Plasmids besteht, welches Insert die bp 1417 bis 2008 des HIV-1 von Ratner et al. (1985, Nature 313: 277-284) enthält. In pgag-15 waren bp1593 bis 1607 deletiert, in pgag+12 war ein Insert von 12 bp an der Stelle 1593 inseriert.

Bei dieser Quantifizierung wurden Primer verwendet, die an die cDNA-Sequenzen von HIV-1 binden und die ein Produkt von 115 (Gew.) bp mittels RT-PCR von Wildtyp-RNA ergeben, nämlich: SK38: ATAATCCACCTATCCCAGTAGGAGAAAT HIV-1 1551-1578 und SK39: TTTGGTCCTTGTCTTATGTCCAGAATGC HIV-1 1665-1638 (Numerierung gemäß Ratner et al.). Die Länge der RT-PCR-Produkte der Standard- und der Wildtyp-DNA war daher 127 (pgag+12) und 100 (pgag-15). Die Primer wurden durch Verwendung der Phosphoamidit-Chemie auf einem DNA-Synthesizer (Applied Biosystems 394 DNA Synthesizer) erzeugt.

Der RT-PCR-Aufbau enthielt auf bekannte Weise ein Aliquot der extrahierten Nucleinsäure, RT-Puffer von Perkin-Elmer, MgCl₂, dNTPs, den RT-Primer und rT.th.Polymerase (Perkin Elmer, 2,5 E/µl) und Wasser. Die RT-PCR erfolgte gemäß der Vorschriften des Herstellers von Puffer und Enzym bzw. gemäß der allgemeinen Arbeitsvorschriften (Mullis et al., Methods in Enzymology 155 (1987), 335-350) in einer PCR-Vorrichtung (GeneAmp PCR System 9600 von Perkin-Elmer).

Zur Bestimmung und Quantifizierung der PCR-Produkte wurden 0,5 bis 1,0 µl der PCR-Lösung genommen und in einem Genescanner 373A-Instrument von Applied Biosystems gemäß den Instruktionen der Erzeuger analysiert.

Die RNA-Kopienzahl der HIV-Virus-Präparationen wurden vor und nach der Behandlung mit Methylenblau/Laserstrahl bestimmt, und die Ergebnisse sind in Tabelle 5 zusammengefaßt.

**Tabelle 5:**

| **Vergleich der Desintegration von HIV-1-Nucleinsäure mittels Methylenblau/Laserstrahl- und Formalin-Behandlung durch Bestimmung von Virustiter und RNA-Kopienzahl** | | |
|---|---|---|
| Behandlung | Virus-Titer (TCID₅₀/ml) | HIV-1-RNA (Kopienzahl/ml) |
| Virus-Stammlösung | 10^{7,6} | 10¹⁰ |
| Virus-Stammlösung mit MB | 10^{7,1} | 10^{9,8} |
| 10 x Zyklen Laser mit MB | <10^{0,3} | <10^{2,6} |
| 20 x Zyklen Laser mit MB | <10^{0,3} | <10^{2,6} |
| 30 x Zyklen Laser mit MB | <10^{0,3} | <10^{2,6} |
| Formaldehyd | <10^{0,3} | 10^{8,3} |

### Beispiel 6 : Desintegration von HSV-DNA und CEC-DNA mittels Methylenblau/Laserstrahl-Behandlung

Ein HSV-1-Glycoprotein-Untereinheit-Vakzin wurde aus mit HSV-1 infizierten Hühnerembryozellen durch Detergens-Extraktion, Saccharose-Gradienten-Reinigung und nachfolgende Lentil-Lectin-Chromatographie hergestellt. Diese Präparation wurde dann einer Formalin-Inaktivierung (30 h, 0,05% Formalin Endkonzentration bei 37°) oder einer Methylenblau/Laserstrahl-Behandlung mit 30 Zyklen mit einer Methylenblau-Endkonzentration von 2 µM unterzogen. Hühnerembryozellen- und HSV-1-DNA in der mit Methylenblau/Laserstrahl und mit Formalin behandelten Probe wurde vor und nach beiden Behandlungen bestimmt.

Für die HSV-DNA-Quantifizierung wurden Primer verwendet, welche im Genom von HSV binden und ein Produkt von 114 bp mittels PCR von Wildtyp-DNA ergeben, nämlich gDR1/B:

AAC TAC CCC GAT CAT CAG und gDR2/B: AGG CCC ACT TAG ACG ACA.

Das Standard-Plasmid pHerp-9 stammte vom Plasmid pHerp, welches aus dem bekannten pCRII-Plasmid (von In Vitrogen) und einem Insert von bp 128364 bis 138784 des HSV-Genoms (McGeoch D.J. et al., EMBL GenBank ID HE1CG1) besteht. 9 bp waren in pHerp-9 deletiert. Die Plasmide pHerp und pHerp-9 wurden gereinigt, die Konzentration wurde durch spektroskopische Messung bei 260 nm bestimmt, und es wurde mit einem Restriktionsenzym linearisiert und in einem TE-Puffer (10 mM Tris/HCl, 1 mM EDTA, pH 8) verdünnt.

Diese DNA-Präparation dient als Standard für die PCR. Die Längen der PCR-Produkte von Standard und Wildtyp waren so 105 (pHerp-9) und 114 bp (pHerp).

Die chromosomale Hühner-DNA wurde unter Verwendung des Primer-Paares CR1: ATGAGGCACTGGAACAGGTTGCCC und CR1A: AGGGCCACATCCAGCCTGG quantifiziert. Diese Primer sind spezifisch für repetitive, von Vögeln stammende Sequenzen, insoferne als sie spezifisch innerhalb der konservierten Sequenzen binden und ein DNA-Fragment mit einer Länge von 846 bp amplifizieren. Die Standard-Plasmide pCR1+11 und pCR1-8 wurden durch Insertion synthetischer Oligonucleotide, welche die von CR1 stammende Sequenz von Position 260 bis 345 (gemäß Stumph et al., 1981, Nucl. Acid. Res. 9: 5383-5397) enthielten, zwischen die NcoI- und SacI-Stellen des p-Bluscript-Vektors, erhalten. Bei pCR1+11 enthält die CR1- Sequenz eine Insertion von 11 Nucleotiden, und beim Standard-Plasmid pCR1-8 eine Deletion von 8 Nucleotiden an Position 302 (gemäß Stumph et al.). Daher sind die von diesen Standard-Plasmiden stammenden PCR-Produkte 95 bp bzw. 76 bp lang.

Die Ergebnisse der HSV-DNA- und CEC-DNA-Quantifizierung sind in Tabelle 6 zusammengefaßt.

**Tabelle 6:**

| **Quantifizierung von HSV-1- und CEC-Nucleinsäure vor und nach der Desintegration mittels Methylenblau/Laser-Behandlung oder Formalin-Inaktivierung eines HSV-Untereinheit-Vakzins** | | |
|---|---|---|
| | DNA-Gehalt (pg/ml) | |
| | CEC-DNA | HSV-DNA |
| Ausgangsmaterial | 95 | 66 |
| 30 Zyklen Laser/MB | <1 | <1 |
| Formalin | 88 | 48 |

### Beispiel 7 : Vergleich der Desintegration von Vero-Zellen-DNA mittels Methylenblau/Laserstrahl- und Formalin-Behandlung

Ein HIV-1-gp160-Kandidaten-Vakzin wurde von rekombinanten, mit Vaccinia infizierten Vero-Zellen gereinigt. Dies erfolgte mittels Extraktion mit Desoxycholat, gefolgt von Lentil-Lectin und Immunoaffinitätschromatographie, wie in Barrett et al. (AIDS Res. Human Retrov. 5 (1989) 159-171).

Diese Präparation wurde dann einer Formalin-Inaktivierung (30 h, 0,05% Formalin-Endkonzentration, 37°C) unterzogen oder 30 Zyklen einer Methylenblau/Laserstrahl-Behandlung mit einer Methylenblau-Endkonzentration von 2 µM, wie in Beispiel 1 beschrieben, unterzogen. Der Vero-Zellen-DNA-Gehalt des Ausgangsmaterials und nach Inaktivierung durch Formalin- und Methylenblau/Laserstrahl-Behandlung wurde unter Verwendung der Primer: Alu A2/2: GCCGGGCGTAGTGGCGGGCGCCTGTAGT und Alu B:  GAGACAGAGTCTCGCTCTGTCGCCCAGG, welche in einem stark konservierten Bereich in den sogenannten "Alu repeat"-Sequenzen binden und ein 146 bp-Fragment (Jelinek et al., Ann. Rec. Biochem. 51 (1982) 813-844) amplifizieren, bestimmt.

Das Standard-Plasmid pAlu20 stammt vom Plasmid pAlu-wt, welches aus dem bekannten pCRII-Plasmid (von InVitrogen) und einem Insert an der multiplen Klonierungsstelle des pCRII-Plasmids besteht, welches Insert die bp 148 bis 294 der Alu-repeat-spezifischen Sequenz von Batzer et al. (1990, Nucl. Acid. Res. 18:6793-6798) enthält. In pAlu20 waren die bp 178 bis 197 deletiert.

Die Längen der PCR-Produkte von Standard pAlu20 und Wildtyp-DNA sind somit 126 bzw. 146 bp.

Die Ergebnisse der DNA-Quantifizierung vor und nach der Desintegration sind in Tabelle 7 zusammengefaßt.

**Tabelle 7:**

| **Desintegration von Vero-Zellen-DNA mittels Methylenblau/Laserstrahl-Behandlung in einem rekombinanten HIV-1 GP160- Vakzin** | |
|---|---|
| | DNA-Gehalt (pg/ml) |
| Ausgangsmaterial | 76 |
| 30 Zyklen Laser/MB | <10 |
| Formalin | 69 |

### Beispiel 8 : Wirkung von Methylenblau/Laserstrahl- Behandlung auf rekombinante, von FVIII stammende Zellkultur

Eine Zellkultur von rekombinanten CHO-Zellen, die mit einer für FVIII-Aktivität codierenden cDNA transfektiert wurden, wurde mit einem ecotropen Mäuse-Retrovirus, Stamm MuLV, versetzt, und Methylenblau wurde zum Erhalt einer Endkonzentration von 2 µM zugegeben. Diese wurde dann 10, 20 und 30 Zyklen einer Laserstrahl-Behandlung, wie in Beispiel 1 beschrieben, unterzogen. Virustiter und FVIII-Aktivität wurden vor der Zugabe von Methylenblau und nach 10, 20 und 30 Zyklen Laserstrahl-Behandlung bestimmt. Der Virustiter wurde unter Verwendung des XC-Plaque-Tests, wie von Hartley et al. (1975, Virology 65, 128-134) beschrieben, bestimmt, und die FVIII-Aktivität wurde nach dem chromogenen Verfahren wie in den Instruktionen für das Immunochrom®-Reagens-Set beschrieben, bestimmt.

Die in Tabelle 8 zusammengefaßten Ergebnisse zeigen, daß das Virus nach 10 Zyklen Laserstrahl-Bestrahlung in Anwesenheit von 2 µM Methylenblau effizient inaktiviert ist. Unter diesen Bedingungen ist die FVIII-Aktivität im wesentlichen unverändert.

**Tabelle 8:**

| **Inaktivierung von MuLV mittels Methylenblau/Laserstrahl-Behandlung in einer rFVIII-Präparation und Bestimmung der rFVIII-Aktivität** | | |
|---|---|---|
| MB/Laserstrahl-Zyklen | FVIII-Aktivität (Einheiten/ml) | MuLV-Titer (FFE/ml) |
| 0 | 21,7 | 10^{5,4} |
| 10 | 20,3 | <10⁰ |
| 20 | 15,9 | <10⁰ |
| 30 | 15,4 | <10⁰ |

### Beispiel 9 : Wirkung von Methylenblau/Laserstrahl-Behandlung auf aus einer Zellkultur stammenden, rekombinanten vWF

Ein Überstand aus einer CHO-(Chinese hamster ovary)-Zellkultur, die rekombinanten vWF exprimiert, wurde einer Methylenblau/Laserstrahl-Behandlung, wie für rFVIII in Beispiel 8 beschrieben, unterzogen. Proben wurden für RistoCo:F-Aktivitäts- und vWF-Antigen-Bestimmung vor der Zugabe von Methylenblau und nach 10, 20 und 30 Zyklen einer Laserstrahl/Methylenblau-Behandlung genommen. Die Ergebnisse sind in Tabelle 9 zusammengefaßt.

**Tabelle 9:**

| **RistoCo:F-Aktivität von rvWF nach Methylenblau/Laser-Behandlung** | |
|---|---|
| MB/Laserlicht-Zyklen | RistoCo:F/vWF Ag |
| 0 | 17,8 |
| 10 | 17,6 |
| 20 | 15,5 |
| 30 | 15,4 |

### Beispiel 10 : Onkogen-Desintegration durch Methylenblau/Laserstrahl -Behandlung

Eine Onkogen-Aktivierung, die zu einem Thymus-Lymphom bei C57 B1/6J-Mäusen führte, wurde durch i.p.-Injektion des chemischen Carcinogens N-Nitrosomethylharnstoff (30 mg pro kg, einmal pro Woche, 5 Wochen hintereinander) induziert. DNA mit hohem Molekulargewicht, die die induzierten Onkogene enthielt, wurde aus Thymus-Tumoren mittels Standard-Techniken hergestellt. Diese wurde mit einer Konzentration von 100µg/ml mit 2 µmol/ml Methylenblau zubereitet und durch Einwirkung von 30 Zyklen Laserlichtstrahl bei 632 nm mit einer Intensität von 10 mW inaktiviert. Kontroll-Präparationen ohne Methylenblau wurden auf identisch Weise behandelt. Die Tumorogenität der beiden Präparationen wurde durch Transfektion-Transformations-Tests auf NIH/3T3-Zellen analysiert. Diese Zellen wurden mit Methylenblau/Laserstrahl-desintegrierter und Kontroll-DNA transfektiert, wobei die CaPO₄-Ausfällungsmethode verwendet wurde. Die ausgefällte DNA wurde mit Gewebskulturmedium gemischt und in Kunststoff-Petrischalen gegeben, die NIH/3T3-zellen (25µg DNA zu 5 x 10⁵ Zellen) enthielten. Nach Subkultivierung und 20tägiger Inkubation wurden zahlreiche große Foci, bestehend aus morphologisch transformierten Zellen, beobachtet und für jede DNA-Präparation in insgesamt zehn Platten gezählt.

**Tabelle 10:**

| **Bestimmung der Onkogenität von DNA vor und nach Laserbehandlung und Methylenblau/Laser-Behandlung** | |
|---|---|
| Onkogen-DNA-Präparation | Anzahl der Foci transformierter Zellen |
| Unbehandelte Kontrolle | 109 |
| mit Laserstrahl-behandelt, ohne Methylenblau | 79 |
| mit Methylenblau behandelt, ohne Laserstrahl | 86 |
| Laserstrahl mit Methylenblau-Behandlung | 4 |

### Beispiel 11 : Onkogen-Desintegration durch Laserstrahl-Behandlung

DNA mit hohem Molekulargewicht, welche induzierte Onkogene enthielt, wurde hergestellt, wie in Beispiel 10 beschrieben, und mit 30 Zyklen Laserstrahl-Einwirkung bei 320 nm mit einer Intensität von 10 mW desintegriert. Die Tumorogenität der Präparation wurde, wie in Beispiel 11 beschrieben, analysiert.

**Tabelle 11:**

| **Bestimmung der Onkogenität von DNA vor und nach Laserbehandlung bei 320 nm** | |
|---|---|
| Onkogen-DNA-Präparation | Anzahl der Foci transformierter Zellen |
| Unbehandelte Kontrolle | 109 |
| mit Laserstrahl behandelt, ohne Methylenblau | 10 |
| mit Methylenblau behandelt, ohne Laserstrahl | 86 |
| Laserstrahl mit Methylenblau-Behandlung | 10 |

### Beispiel 12 : Inaktivierung von Mäuse-Retroviren in einem monoklonale Antikörper enthaltenden Hybridom-Überstand

Ein ecotroper Mäuse-Retrovirus, MuLV Stamm SN-E81, wurde einem Hybridom-Überstand, welcher einen gegen HIV-1 gp160 gerichteten monoklonalen Antikörper enthielt, zugesetzt. Methylenblau wurde dem Überstand bis zu einer Endkonzentration von 2 µM zugegeben, und die Mischung wurde, wie oben beschrieben, 10, 20 und 30 Zyklen einer Laserstrahlbehandlung unterzogen. Der Virus- und Antikörper-Titer wurde vor der Zugabe von Methylenblau und nach 10, 20 und 30 Zyklen Laserstrahl-Behandlung bestimmt. Der Virus-Titer wurde unter Verwendung des XC-Focusbildungstests, wie von Hartley et al., 1975 beschrieben, bestimmt. Virology 65, 128-134. Der Antikörper-Titer wurde mittels Standard-ELISA-Techniken bestimmt, wobei mit rekombinantem gp160 beschichtete Platten verwendet wurden. Kurz gesagt wurden die Mikrotiterplatten mit 0,5 µg gereinigtem, rekombinantem HIV-1 gp160 beschichtet, wobei 100 µl pro Vertiefung verwendet wurden. Die Seren wurden seriell zweifach verdünnt, und 100 µl jeder Verdünnung wurden in die vorbeschichteten Platten gegeben und 1 h lang bei 37°C inkubiert. Nach gründlichem Waschen wurde jede Vertiefung mit 100 µl von Peroxidase konjugiertem anti-Maus-IgG gefüllt und 1 h lang bei 37°C inkubiert. Nach dem Waschen wurden 200 µl aktiviertes Substrat (O-Phenylendiamin-Hydrochlorid) in jede Vertiefung zugegeben und im Dunklen 10 Minuten lang bei Raumtemperatur inkubiert. Die Reaktion wurde durch die Zugabe von 50 µl 5M Schwefelsäure gestoppt, und die Extinktion wurde bei 490 nm abgelesen. Der Antikörper-Titer wurde als reziproker Wert der höchsten Verdünnung, die eine positive Reaktion ergab, bestimmt.

Die Ergebnisse sind in Tabelle 12 zusammengefaßt.

**Tabelle 12:**

| **MuLV-Titer und Antikörper-Titer nach Methylenblau/ Laser-Behandlung** | | |
|---|---|---|
| MB/Laserstrahl-Zyklen | MuLV-Titer | Antikörper-Titer |
| 0 | 10^{5,3} | 1:5120 |
| 10 | <10⁰ | 1:5120 |
| 20 | <10⁰ | 1:5120 |
| 30 | <10⁰ | 1:2560 |

### Beispiel 13 : Wirksamkeit von mit Formalin und mit Methylenblau/Strahlenlicht inaktiviertem TBEV in einem Maus-Challenge-Modell

Auf Hühnerembryozellen gezüchtetes Tick-Borne Encephalitis-Virus (TBEV) wurde entweder durch Formalin-Behandlung (0,05% Formalin, 37°C, 30 h lang) oder durch Methylenblau-Behandlung (30 Zyklen Laserstrahlen mit einer Methylenblau-Konzentration von 2 µM) inaktiviert. Beide Präparationen wurden mittels Saccharose-Gradienten-Zentrifugation, gefolgt von Dialyse, weiter gereinigt. Die Proben wurden verdünnt, um gleiche Proteinkonzentrationen zu erhalten.

Die Immunogenität der Präparationen wurde mittels PD₅₀-Bestimmung, gefolgt von Adjuvantierung mit Alaun, bestimmt. Gruppen von zwanzig Mäusen wurden subkutan mit dreifachen Verdünnungen der adjuvantierten Vakzin-Präparationen immunisiert. Eine Woche nach der Immunisierung erhielten die Mäuse einen Booster mit derselben Antigen-Dosis. Zwei Wochen nach der zweiten Immunisierung wurden die Mäuse einem Challenge durch i.p.-Impfung mit 10² LD₅₀-Dosen des lebenden Virus unterzogen. Die Mäuse wurden dann über einen Zeitraum von 21 Tagen beobachtet, und die Anzahl der nach diesem Zeitraum überlebenden Mäuse wurde aufgezeichnet. Die Schutzdosis für 50% der Mäuse (PD₅₀) wurde gemäß dem Verfahren von Kärber, 1931, Arch. Exp. Pathol. Pharmakol. 162, 480-483, berechnet und ist in Tabelle 13 gezeigt.

**Tabelle 13:**

| **Wirksamkeitsmessung von mit Methylenblau/Laserstrahl und mit Formalin inaktivierten TBEV-Vakzinen** | |
|---|---|
| Vakzin-Präparation | PD₅₀ (ng) |
| mit Formalin inaktiviert | 32,3 |
| mit Laserstrahl/MB inaktiviert | 18,7 |

### Beispiel 14 : Vergleich der Reaktivität von mit Formalin inaktiviertem und mit Methylenblau/Laserstrahl inaktiviertem Tick-Borne Encephalitis-Virus bei einer Serie spezifischer monoklonaler Antikörper

TBE-Virus wurde auf primären Hühnerembryozellen gezüchtet und mit zwei Zyklen Saccharose-Dichtegradientenzentrifugation gereinigt. Es wurde dann mittels Ultrazentrifugation pelletiert, in 10 mM Tris-HCl, pH 7,2, resuspendiert und eine Formalin-Inaktivierung (0,05% Formalin Endkonzentration, 30h bei 37°C) oder Methylenblau/Laserstrahl-Inaktivierung (2 µM Methylenblau-Endkonzentration, zehn Zyklen) vorgenommen. Beide Präparationen wurden dann einer Sephadex 6-25-Chromatographie unterzogen und nach der Elution eingestellt, um gleiche Proteinkonzentrationen zu erhalten.

Die Reaktivitäten beider Präparationen mit acht verschiedenen monoklonalen Antikörpern, die gegen TBE-Virus-Glycoprotein gerichtet waren, wurden mittels Standard-ELISA-Techniken bestimmt. Mikrotiterplatten wurden mit der Viruspräparation mit einer Konzentration von 2 µg/ml in Carbonatpuffer, pH 9,6, beschichtet. Nach dem Waschen wurden die monoklonalen Antikörper zugegeben und 1 h lang bei 37°C reagieren lassen. Peroxidase-konjugiertes anti-Maus-Immunoglobulin wurde dann zugegeben, und nach einstündiger Inkubation bei 37°C und Waschen wurde das Substrat zugegeben, und die Extinktion wurde bei 492 nm gemessen, nachdem die Reaktion durch Zugabe von 2N H₂SO₄ gestoppt worden war. Alle monoklonalen Antikörper zeigten eine höhere Reaktivität bei der mit Methylenblau/Laserstrahl inaktivierten Virus-Präparation als bei der mit Formalin inaktivierten Vakzin-Präparation. Die Ergebnisse sind in Tabelle 14 zusammengefaßt.

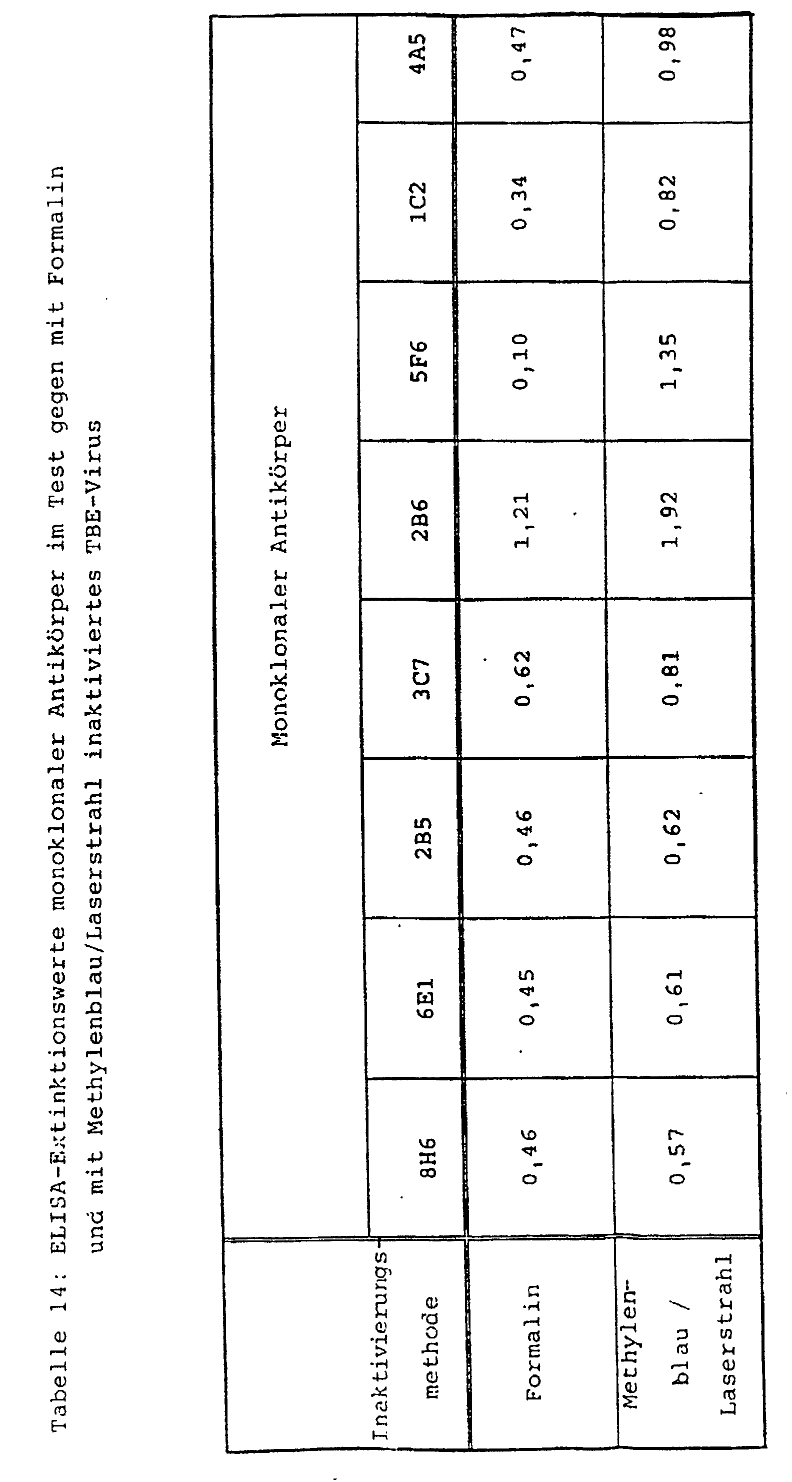

### Beispiel 15 : Desintegration von Nucleinsäure eines prokaryontischen Pathogens durch Methylenblau/Laserstrahl-Behandlung

Eine Kultur von B.burgdorferi-Zellen wurde bis zu einer Zellzahl von 1 x 10⁷ Spirochäten/ml gezüchtet, und Methylenblau wurde bis zu einer Endkonzentration von 2 µM zugegeben. Die Kultur wurde 30 Zyklen einer Laserstrahlbehandlung unterzogen. Die Anwesenheit von B.burgdorferi-DNA vor und nach der Behandlung mit Methylenblau/Laserstrahl wurde mittels PCR-Amplifikation unter Verwendung der Primer LD1: ATG CAC ACT TGG TGT TAA CTA LD2: GAC TTA TCA CCG GCA GTC TTA (gemäß Marconi et al., 1992, J. Clin Microbiol. 30:2830-2834) bestimmt.

### Beispiel 16 : Inaktivierung von Parvovirus in einem von Humanplasma stammenden Produkt mittels Methylenblau/Laserstrahl-Behandlung

Eine hochreine FVIII-Konzentratlösung wurde mit Parvovirus Minute-Mäuse-Virus (Minute Virus of Mice, MVM) versetzt und Methylenblau bis zu einer Endkonzentration von 30 µM zugegeben. Diese wurde dann 10, 20 und 30 Zyklen einer Laserstrahl-Behandlung, wie in Beispiel 1 beschrieben, unterzogen. Die Virus-Titer und die FVIII-Aktivität wurden vor der Zugabe von Methylenblau und nach 10, 20 und 30 Zyklen Laserstrahl-Behandlung bestimmt. Der Virus-Titer wurde mittels TCID₅₀-Bestimmung (Reed et al., 1938, Amer. J. Hyg. 27:493-497) unter Verwendung von A9-Zellen (ATCC CRL 6319) gemessen, und die FVIII-Aktivität wurde gemäß dem chromogenen Verfahren, wie in den Instruktionen für das Immunochrom-Reagens-Set beschrieben, bestimmt.

Die in Tabelle 15 zusammengefaßten Ergebnisse zeigen, daß fast 5 log MVM nach 30 Zyklen Laserstrahl/Methylenblau-Behandlung inaktiviert sind. Unter diesen Bedingungen bleiben mehr als 50% der FVIII-Aktivität erhalten.

**Tabelle 15:**

| **Inaktivierung von MVM-Parvovirus in FVIII-Konzentrat durch Methylenblau/Laserstrahl-Behandlung** | | |
|---|---|---|
| Laserstrahl-Zyklen | MVM-Titer (TCID₅₀/ml) | FVIII-Aktivität (Einheiten/ml) |
| 0 | 10^{7,2} | 49,7 |
| 10 | 10^{5,2} | 41,6 |
| 20 | 10^{3,6} | 31,8 |
| 30 | 10^{2,5} | 25,6 |

### Beispiel 17 : Desintegration der Nukleinsäure von B19 Parvovirus durch Methylenblau/Laser-Behandlung und Nachweis der Desintegration

Eine Plasmapräparation wurde mit einer hochtitrigen Virusstocklösung von humanem Parvovirus B19 beaufschlagt. Zu dieser Mischung wurde Methylenblau bis zu einer Endkonzentration von 30 µM zugegeben. Nach gründlichem Mischen der Lösung wurde das Material durch ein Röhrchen aus rostfreiem Stahl mit einem Glasfenster mit einem Durchmesser von 2 x 2 mm mit einer Durchflußrate von 4 ml/min gepumpt. Das Glasfenster wurde dabei mit einem Laserstrahl eines He-Ne-Laser bei einer Wellenlänge von 633 nm und einer Laserleistung von 10 mM bestrahlt. Das Material wurde zwischen 0 und 50 Zyklen bestrahlt und in Proben jeweils nach O, 1, 5, 10, 20, 30, 40 und 50 Zyklen die B19 Parvovirus-DNA quantitativ mittels PCR bestimmt. Als Kontrolle diente eine Mischung enthaltend Methylenblau, die jedoch nicht mit Laserlicht bestrahlt wurde. Die Ergebnisse der quantitativen Bestimmung der B19-spezifischen DNA Kopienzahl sind in Tabelle 16 zusammenfaßt. Die Ergebnisse zeigen, daß nach etwa 40 Zyklen mit Methylenblau/Laserlicht-Behandlung der Gehalt an B19-spezifischer DNA bis unterhalb der Nachweisgrenze des Meßsystems reduziert war.

**Tabelle 16:**

| **Desintegration von Parvovirus B19 DNA mittels Methylenblau/Laserstrahlbehandlung und Bestimmung der DNA-Kopienzahl vor und nach Behandlung** | |
|---|---|
| Behandlung | B19 DNA-kopienzahl pro ml |
| Keine | 10^{11.4} |
| 1 × Zyklus MB/Laser | 10^{9.6} |
| 5 × Zyklen MB/Laser | 10^{7.8} |
| 10 × Zyklen MBlLaser | 10^{6.0} |
| 20 × Zyklen MB/Laser | 10^{4.6} |
| 30 × Zyklen MB/Laser | 10^{3.0} |
| 40 × Zyklen MB/Laser | <10^{2.7} |
| 50 × Zyklen MB/Laser | <10^{2.7} |
| 50 Zyklen mit MB ohne Laser | 10^{8.9} |

## Patentansprüche

1. Verfahren zur Desintegration von Nukleinsäuren in einem biologisch aktiven protein-haltigen Material, **dadurch gekennzeichnet, daß** einem biologischen Material eine photodynamische Substanz, ausgewählt aus der Gruppe der Phenothiazine, zugesetzt wird, die Mischung einer Bestrahlung mit Laserlicht mit einer Intensität von ≤ 0,1 J/cm² pro Zyklus ausgesetzt wird, um die Nukleinsäure-Template-Aktivität im biologischen Material um mindestens einen Faktor von 7 log-Stufen bzw. den Gehalt an Nukleinsäure auf <100 pg/ml, vorzugsweise <10 pg/ml, besonders bevorzugt <1 pg/ml zu verringern, um im wesentlichen die gesamte biologisch aktive Nukleinsäure in diesem biologischen Material zu desintegrieren bzw. zu inaktivieren, während die biologische Integrität und Aktivität des biologischen Materials im wesentlichen erhalten bleibt.

2. Verfahren nach Anspruch 1, **dadurch gekennzeichnet, daß** die Inaktivierung bzw. Desintegration der Nukleinsäure durch ein Verfahren zur Quantifizierung von Nukleinsäuren kontrolliert wird und ein biologisches Material erhalten wird, dessen Gehalt an Nukleinsäure unterhalb von 100 pg, vorzugsweise von 10 pg, besonders bevorzugt von 1 pg pro Dosis bzw. maximal 1 bis 500 Kopien/ml aufweist und und damit im wesentlichen frei von Nukleinsäure-Template-Aktivität ist.

3. Verfahren nach Anspruch 1 oder 2, **dadurch gekennzeichnet, daß** die photodynamische Substanz Methylenblau, Toluidinblau oder Azur B ist.

4. Verfahren nach einem der Ansprüche 1 bis 3, **dadurch gekennzeichnet, daß** die photodynamische Substanz in einer Konzentration zwischen 0,01 µM und 50 µM, vorzugsweise zwischen 1 µM und 40 µM, vorliegt.

5. Verfahren nach einem der Ansprüche 1 bis 4, **dadurch gekennzeichnet, daß** die Wellenlänge des Laserstrahls im Wellenbereich der maximalen Aktivierungsrate der photodynamischen Substanz ist, vorzugsweise zwischen 600 und 680 nm.

6. Verfahren nach einem der Ansprüche 1 bis 5, **dadurch gekennzeichnet, daß** die Intensität des Laserlichte 0,08 J/cm², und besonders bevorzugt ≤ 0,06 J/cm² ist.

7. Verfahren nach einem der Ansprüche 1 bis 6, **dadurch gekennzeichnet, daß** vor der Laser-Bestrahlung ein Stabilisator, wie Zucker, Polyalkohol, Aminosäuren, Peptide oder Carbonsäuren, und/oder ein Quencher und/oder Scavenger, wie etwa Mannit, Glycerin, reduziertes Glutathion oder Superoxid-Dismutase zugegeben wird.

8. Verfahren nach einem der Ansprüche 1 bis 7, **dadurch gekennzeichnet, daß** vor der Zugabe der photodynamischen Substanz ein Quencher, ein Scavenger und/oder ein Stabilisator zugegeben werden.

9. Verfahren nach einem der Ansprüche 1 bis 8, **dadurch gekennzeichnet, daß** das Verfahren zur Quantifizierung von Nukleinsäuren vorzugsweise ein Nukleinsäureamplifikationsverfahren ist, bei dem vor dem Amplifizierungsschritt eine gegebene Menge mindestens eines bekannten Nukleinsäuremoleküls als interner Standard zugegeben wird, wobei sich das Standard-Molekül von der zu quantifizierenden Nukleinsäure in mindestens einem detektierbaren Merkmal unterscheidet.

10. Verfahren nach einem der Ansprüche 1 bis 9, **dadurch gekennzeichnet, daß** das biologisch aktive Material eine Lösung ist, welche einen Blutfaktor, ein gentechnisch hergestelltes proteinhaltiges Produkt oder ein mikrobiologisches und/oder molekulares Pathogen oder einen Teil davon aufweist.

11. Verfahren nach einem der Ansprüche 1 bis 10, **dadurch gekennzeichnet, daß** das biologische Material eine Zellkultur, eine Zellsuspension, eine Zellschicht, ein Zellkulturüberstand oder eine aufgebrochene Zellpräparation ist, die von infizierten, transfektierten oder transformierten Zellen stammt.

12. Verfahren nach einem der Ansprüche 1 bis 11, **dadurch gekennzeichnet, daß** die aktive Nukleinsäure eine DNA oder RNA, ein Genom oder ein Teil davon, wie etwa ein Gen, ist.

13. Verfahren nach einem der Ansprüche 1 bis 12, **dadurch gekennzeichnet, daß** die aktive Nukleinsäure eine Nukleinsäure-Template-Aktivität aufweist und eine Sequenz für Primer-und Polymerase-Bindung inkludiert, wobei die Nukleinsäure mittels eines Nukleinsäure-Amplifikationsverfahrens amplifizierbar ist.

14. Verfahren nach einem der Ansprüche 1 bis 13, **dadurch gekennzeichnet, daß** die aktive Nukleinsäure von einer eukaryotischen Quelle, wie etwa einer Vertebraten-Zelle, einer Tumor-Zellinie, einem Onkogen oder einem Protoonkogen oder einer Hybridoma-Zellinie stammt.

15. Verfahren nach einem der Ansprüche 1 bis 14, **dadurch gekennzeichnet, daß** die Nukleinsäure von einem Mikroorganimus, wie etwa einem Protozoon, einem Bakterium, einem Virus, einem mikrobiologischen oder molekularen Pathogen oder einem Teil davon stammt.

16. Verfahren zur Inaktivierung von Viren, **dadurch gekennzeichnet, daß** einem virushaltigen biologischen Material eine photodynamische Substanz, ausgewählt aus der Gruppe der Phenothiazine, zugesetzt wird, die Mischung einer Bestrahlung mit Laserlicht mit einer Intensität von ≤ 0,1 J/cm² pro Zyklus ausgesetzt wird, um die Nukleinsäure-Template-Aktivität im biologischen Material um mindestens einen Faktor von 7 log-Stufen bzw. der Gehalt an Nukleinsäure auf <100 pg/ml, vorzugsweise <10 pg/ml, besonders bevorzugt <1 pg/ml zu verringern, um im wesentlichen die gesamte biologisch aktive Nukleinsäure in diesem biologischen Material zu desintegrieren bzw. zu inaktivieren, während die biologische Integrität und Aktivität des biologischen Materials im wesentlichen erhalten bleibt.

17. Verfahren nach Anspruch 16, **dadurch gekennzeichnet, daß** das Virus ein Lipid-(umhülltes), ein nicht-Lipid-(umhülltes) Virus, ein DNA- oder ein RNA-Virus oder ein attenuiertes Virus ist.

18. Verfahren nach Anspruch 17, **dadurch gekennzeichnet, daß** das Virus, ausgewählt aus der Gruppe Adenovirus, Herpesvirus, Papoavirus, Poxvirus, Parvovirus, Reovirus, Retrovirus, Myxovirus, Paramyxovirus, Picornavirus, Togavirus, Plavivirus, Orthomyxovirus oder Rhadovirus, vorzugsweise ein Influenzavirus, HIV, HCV, HAV, HBV, TBEV oder CMV ist, wobei das Virus gegebenenfalls attenuiert ist.

19. Verfahren zur Inaktivierung von nicht-umhüllten Viren in einem proteinhaltigen biologischen Material, **dadurch gekennzeichnet, daß** einem biologischen Material eine photodynamische Substanz, ausgewählt aus der Gruppe der Phenothiazine, in einer Konzentration von mindestens 20 µM zugesetzt wird und die Mischung einem Laserstrahl im Wellenbereich der maximalen Aktivierungsrate der photodynamischen Substanz mit einer Intensität des Laserlichtes von ≤ 0,1 J/cm² pro Zyklus, vorzugsweise ≤ 0,06 J/cm² pro Zyklus für mindestens 20 Zyklen ausgesetzt wird.

20. Verfahren nach Anspruch 19, **dadurch gekennzeichnet, daß** nicht-umhüllte Viren, insbesondere aus der Gruppe der Picornaviridae und Parvoviridae inaktiviert werden.

21. Verfahren nach Anspruch 19 oder 20, **dadurch gekennzeichnet, daß** im wesentlichen die gesamte biologisch aktive, infektiöse Nukleinsäure in diesem biologischen Material desintegriert bzw. inaktiviert wird, während die biologische Integrität und Aktivität des protein-haltigen biologischen Materials erhalten bleibt.

22. Verfahren nach Anspruch 21, **dadurch gekennzeichnet, daß** die Inaktivierung bzw. Desintegration der Nukleinsäure durch ein Verfahren zur Quantifizierung von Nukleinsäuren kontrolliert wird und ein biologisches Material erhalten wird, dessen Gehalt an Nukleinsäure unterhalb von 100 pg, vorzugsweise von 10 pg, besonders bevorzugt von 1 pg pro Dosis bzw. maximal 1 bis 500 Kopien/ml aufweist und damit im wesentlichen frei von Nukleinsäure-Template-Aktivität ist.

23. Verwendung eines Verfahrens nach einem der Ansprüche 16 bis 22 zur Herstellung eines virus-inaktivierten biologischen Materials, das insbesondere keine infektiösen nicht-Lipidumhüllten Viren aufweist.

24. Verwendung eines Verfahrens nach einem der Ansprüche 16 bis 22 zur Herstellung einer inaktivierten Virus-Vakzine.

25. Verfahren zur Herstellung einer inaktivierten Virus-Vakzine, **dadurch gekennzeichnet, daß** einem virus-haltigen Material eine photodynamische Substanz, ausgewählt aus der Gruppe der Phenothiazine, zugesetzt wird, die Mischung einer Bestrahlung mit Laserlicht mit einer Intensität von ≤ 0,1 J/cm² pro Zyklus ausgesetzt wird, um im wesentlichen die gesamte biologisch aktive Nukleinsäure im biologischen Material zu desintegrieren bzw. zu inaktivieren, während die biologische Integrität und Aktivität, wie Antigenität und Immunogenität des Virus im wesentlichen erhalten bleibt, die Desintegration der Nukleinsäure durch ein Verfahren zur Quantifizifierung von Nukleinsäuren kontrolliert wird und ein virus-haltiges Material erhalten wird, dessen Gehalt an Nukleinsäure unterhalb von 100 pg, vorzugsweise von 10 pg, besonders bevorzugt von 1 pg pro Dosis bzw. maximal 1 bis 500 Kopien/ml aufweist und damit im wesentlichen frei von Nukleinsäure-Template-Aktivität ist, die inaktivierten Viren gegebenenfalls einer weiteren Reinigung unterzogen werden und die inaktivierten Viren mit einem physiologisch akzeptablen Träger zu einer Virus-Vakzine formuliert werden.

26. Verfahren nach Anspruch 25, **dadurch gekennzeichnet, daß** der Mikroorganismus ein virales Pathogen, wie ein Lipid- oder Nichtlipid umhülltes Virus, ein Virus enthaltend DNA oder RNA, ausgewählt aus der Gruppe Adenovirus, Herpesvirus, Papoavirus, Poxvirus, Parvovirus, Reovirus, Retrovirus, Myxovirus, Paramyxovirus, Picornavirus, Togavirus, Flavivirus, Orthomyxovirus oder Rhadovirus, vorzugsweise ein Influenzavirus, HIV, HCV, HAV, HBV, TBEV oder CMV ist, wobei das Virus gegebenenfalls attenuiert ist.

27. Verfahren nach einem der Ansprüche 25 oder 26, **dadurch gekennzeichnet, daß** das virus-haltige Material von einer Kultur von virusinfizierten Zellen, einem Kulturüberstand von virusinfizierten Zellen, einer Zellkomponente von virusinfizierten Zellen oder einer gereinigte Viren enthaltenden Lösung stammt.

28. Verfahren nach Anspruch 27, **dadurch gekennzeichnet, daß** die virusinfizierten Zellen Vertebraten-Zellen, wie Hühnerembryonalzellen, VERO-Zellen, CV-1-Zellen, LLC-MK-2-Zellen, MDCK-Zellen, MDBK-Zellen, WI-38-Zellen oder MRC-5-Zellen, sind.

29. Verfahren nach einem der Ansprüche 25 bis 28, **dadurch gekennzeichnet, daß** die inaktivierte bzw. desintegrierte Nukleinsäure von der Zelle und/oder dem Virus stammt.

## Claims

1. A method for the disintegration of nucleic acids in a biologically active proteinaceous material, **characterised in that** a photodynamic substance selected from the group of phenothiazines is admixed to a biological material, the mixture is exposed to an irradiation with laser light having an intensity of ≤ 0.1 J/cm² per cycle, to respectively reduce the nucleic acid template activity in the biological material at least by a factor of 7 log steps or to reduce the content of nucleic acid to < 100 pg/ml, preferably < 10 pg/ml, particularly preferred < 1 pg/ml so as to disintegrate or inactivate, respectively, essentially all biologically active nucleic acid in this biological material, while the biological integrity and activity of the biological material is substantially maintained.

2. A method according to claim 1, **characterised in that** the inactivation or disintegration, respectively, of the nucleic acid is checked by a method of quantitating nucleic acids and a biological material is obtained whose content of nucleic acid is less than 100 pg, preferably less than 10 pg, particularly preferred less than 1 pg per dose or has 1 to 500 copies/ml at the most, respectively, and thus is substantially free from nucleic acid template activity.

3. A method according to claim 1 or 2, **characterised in that** the photodynamic substance is methylene blue, toluidine blue or azur B.

4. A method according to any one of claims 1 to 3, **characterised in that** the photodynamic substance is present at a concentration of between 0.01 µM and 50 µM, preferably between 1 µM and 40 µM.

5. A method according to any one of claims 1 to 4, **characterised in that** the wave length of the laser beam is in the wave length of the maximal activation rate of the photodynamic substance, preferably between 600 and 680 nm.

6. A method according to any one of claims 1 to 5, **characterised in that** the intensity of the laser light is ≤ 0.08 J/cm², and particularly preferred ≤ 0.06 J/cm².

7. A method according to any one of claims 1 to 6, **characterised in that** prior to exposure to laser beam, a stabiliser, such as sugar, polyalcohol, amino acids, peptides or carboxylic acids, and/or a quencher and/or a scavenger, such as mannitol, glycerol, reduced glutathione or superoxide dismutase is added.

8. A method according to any one of claims 1 to 7, **characterised in that** prior to the addition of the photodanymic substance, a quencher, a scavenger and/or a stabiliser is added.

9. A method according to any one of claims 1 to 8, **characterised in that** the method of quantitating nucleic acids preferably is a nucleic acid amplification method, in which a given amount of at least one known nucleic acid molecule is added as an internal standard prior to the amplification step, the standard molecule differing from the nucleic acid to be quantitated in at least one detectable characteristic.

10. A method according to any one of claims 1 to 9, **characterised in that** the biologically active material is a solution comprising a blood factor, a genetically engineered proteinaceous product or a microbiological and/or molecular pathogen or a part thereof.

11. A method according to any one of claims 1 to 10, **characterised in that** the biological material is a cell culture, a cell suspension, a cell layer, a cell culture supernatant or a broken-up cell preparation derived from infected, transfected or transformed cells.

12. A method according to any one of claims 1 to 11, **characterised in that** the active nucleic acid is a DNA or an RNA, a genome or a part thereof, such as, e.g., a gene.

13. A method according to any one of claims 1 to 12, **characterised in that** the active nucleic acid has nucleic acid template activity and includes a sequence for primer and polymerase binding, the nucleic acid being amplifyable by means of a nucleic acid amplification method.

14. A method according to any one of claims 1 to 13, **characterised in that** the active nucleic acid is derived from a eukaryotic source, such as, e.g., a vertebrate cell, a tumor cell line, an oncogene or a protooncogene or from a hybridoma cell line.

15. A method according to any one of claims 1 to 14, **characterised in that** the nucleic acid is derived from a microorganism, such as, e.g., a protozoon, a bacterium, a virus, a microbiological or molecular pathogen or a part thereof.

16. A method of inactivating viruses, **characterised in that** a photodynamic substance selected from the group of phenothiazines is admixed to a virus-containing biological material, the mixture is exposed to an irradiation with laser light having an intensity of ≤ 0.1 J/cm² per cycle to reduce the nucleic acid template activity in the biological material at least by a factor of 7 log steps or to reduce the content of nucleic acid to < 100 pg/ml, preferably < 10 pg/ml, particularly preferred < 1 pg/ml, respectively, so as to disintegrate or inactivate, respectively, essentially all biologically active nucleic acid in this biological material, while the biological integrity and activity of the biological material is substantially maintained.

17. A method according to claim 16, **characterised in that** the virus is a lipid-(enveloped), a non-lipid-(enveloped) virus, a DNA virus or an RNA virus or an attenuated virus.

18. A method according to claim 17, **characterised in that** the virus, selected from the group of adenovirus, herpes virus, papovavirus, poxvirus, parvovirus, reovirus, retrovirus, myxovirus, paramyxovirus, picornavirus, Toga virus, flavivirus, orthomyxovirus or rhabdovirus, preferably is an influenza virus, HIV, HCV, HAV, HBV, TBEV or CMV, the virus optionally being attenuated.

19. A method of inactivating non-enveloped viruses in a proteinaceous biological material, **characterised in that** a photodynamic substance selected from the group of phenothiazines is admixed at a concentration of at least 20 µM to a biological material, and the mixture is exposed to a laser beam in the wave range of the maximum activation rate of the photodynamic substance the laser light having an intensity of ≤ 0.1 J/cm² per cycle, preferably ≤ 0.06 J/cm² per cycle, for at least 20 cycles.

20. A method according to claim 19, **characterised in that** non-enveloped viruses, in particular from the group of picornaviridae and parvoviridae, are inactivated.

21. A method according to claim 19 or 20, **characterised in that** substantially all the biologically active, infectious nucleic acid in this biological material is disintegrated or inactivated, respectively, while the biological integrity and activity of the proteinaceous biological material is maintained.

22. A method according to claim 21, **characterised in that** the inactivation or disintegration, respectively, of the nucleic acid is checked by a method of quantitating nucleic acids, and a biological material is obtained whose content of nucleic acid is less than 100 pg, preferably less than 10 pg, particularly preferred less than 1 pg per dose or 1 to 500 copies/ml at the most, respectively, and thus is substantially free from nucleic-acid-template activity.

23. The use of a method according to any one of claims 16 to 22 for producing a virus-inactivated biological material which, in particular, does not comprise any infectious non-lipid-enveloped viruses.

24. The use of a method according to any one of claims 16 to 22 for producing an inactivated virus vaccine.

25. A method for recovering an inactivated virus-vaccine, **characterised in that** a photodynamic substance selected from the group of phenothiazines is admixed to a virus-containing material, the mixture is exposed to an irradiation with laser light having an intensity of ≤ 0.1 J/cm² per cycle to disintegrate or inactivate, respectively, essentially all biologically active nucleic acid in the biological material, while the biological integrity and activity, such as antigenicity and immunogenicity, of the virus is substantially maintained, the disintegration of the nucleic acid is checked by a method of quantitating nucleic acids, and a virus-containing material is obtained whose content of nucleic acid is less than 100 pg, preferably less than 10 pg, particularly preferred less than 1 pg per dose or has 1 to 500 copies/ml at the most, respectively, and thus is substantially free from nucleic acid template activity, the inactivated viruses optionally are subjected to a further purification, and the inactivated viruses are formulated with a physiologically acceptable carrier into a virus-vaccine.

26. A method according to claim 25, **characterised in that** the microorganism is a viral pathogen, such as a lipid- or non-lipid-(enveloped) virus, a virus containing DNA or RNA selected from the group of adenovirus, herpes virus, papovavirus, poxvirus, parvovirus, reovirus, retrovirus, myxovirus, paramyxovirus, picornavirus, Toga virus, flavivirus, orthomyxovirus or rhabdovirus, preferably an influenza virus, HIV, HCV, HAV, HBV, TBEV or CMV, the virus optionally being attenuated.

27. A method according to any one of claims 25 or 26, **characterised in that** the virus-containing material is derived from a culture of virus-infected cells, a culture supernatant of virus-infected cells, a cell component of virus-infected cells or a solution comprising purified viruses.

28. A method according to claim 27, **characterised in that** the virus-infected cells are vertebrate cells, such as chicken embryo cells, VERO cells, CV-1 cells, LLC-MK2 cells, MDCK cells, MDBK cells, WI-38 cells or MRC-5 cells.

29. A method according to any one of claims 25 to 28, **characterised in that** the inactivated or disintegrated, respectively, nucleic acid is derived from the cell and/or from the virus.

## Revendications

1. Procédé de désintégration d'acides nucléiques dans un matériau protéinique biologiquement actif, **caractérisé en ce que** l'on ajoute à un matériau biologique une substance photodynamique, choisie dans le groupe des phénothiazines, le mélange étant soumis à une irradiation à la lumière laser avec une intensité de < 0,1 J/cm² par cycle, pour réduire l'activité du modèle d'acide nucléique dans le matériau biologique d'un facteur correspondant à au moins 7 échelons logarithmiques, respectivement la teneur en l'acide nucléique à < 100 pg/ml, de préférence à < 10 pg/ml, encore mieux à < 1 pg/ml, pour désintégrer ou inactiver pour l'essentiel le total de . l'acide nucléique biologiquement actif dans ce matériau biologique, tout en conservant pour l'essentiel l'intégrité et l'activité biologique du matériau biologique.

2. Procédé selon la revendication 1, **caractérisé en ce que** l'inactivatio ou la désintégration de l'acide nucléique est contrôlée par un procédé de quantification des acides nucléiques et qu'un matériau biologique est obtenu, dont la teneur en acide nucléique est inférieure à 100 pg, de préférence à 10 pg, encore mieux à 1 pg par dose, respectivement présente au maximum 1 à 500 copies/ml et est ainsi essentiellement exempt de l'activité de modèle de l'acide nucléique.

3. Procédé selon l'une des revendications 1 ou 2, **caractérisé en ce que** la substance photodynamique est du bleu de méthylène, du bleu de toluidine ou de l'azur B.

4. Procédé selon l'une des revendications 1 à 3, **caractérisé en ce que** la substance photodynamique est présente à une concentration comprise entre 0.01 µM et 50 µM, de préférence entre 1 µM et 40 µM.

5. Procédé selon l'une des revendications 1 à 4, **caractérisé en ce que** la longueur d'onde du faisceau laser est comprise dans le domaine d'ondes de l'activation maximale de la substance photodynamique, de préférence 600 à 680 nm.

6. Procédé selon l'une des revendications 1 à 5, **caractérisé en ce que** l'intensité de la lumière laser est ≤ 0,08 J/cm², et de préférence ≤ 0,06 J/cm².

7. Procédé selon l'une des revendications 1 à 6, **caractérisé en ce qu'**un stabilisateur, tel le sucre, un polyol, des acides aminés, des peptides ou des acides carboxyliques, un agent de trempe (Quencher) et / ou de balayage (Scavenger) comme du mannitol, de la glycérine, du glutathion réduit ou une dismutase de superoxyde sont ajoutés avant l'irradiation par le laser.

8. Procédé selon l'une des revendications 1 à 7, **caractérisé en ce qu'**avant l'addition de la substance photodynamique, un agent de trempe (Quencher), de balayage (Scavenger) et / ou un stabilisateur sont ajoutés.

9. Procédé selon l'une des revendications 1 à 8, **caractérisé en ce que** le procédé de quantification des acides nucléiques est de préférence un procédé d'amplification de l'acide nucléique, pour lequel, avant l'étape d'amplification, une quantité donnée d'au moins une espèce moléculaire connue d'un acide nucléique est ajoutée comme standard interne, la molécule standard se distinguant de l'acide nucléique à quantifier par au moins une caractéristique détectable.

10. Procédé selon l'une des revendications 1 à 9, **caractérisé en ce que** le matériau biologiquement actif est une solution qui présente un facteur hématologique, un produit protéinique obtenu par génie génétique ou un pathogène microbiologique et / ou moléculaire, ou une partie de ceux-ci.

11. Procédé selon l'une des revendications 1 à 10, **caractérisé en ce que** le matériau biologique est une culture cellulaire, une suspension de cellules, une couche de cellules, un reste de culture cellulaire ou une préparation de cellules lysées, qui provient de cellules infectées, transfectées ou transformées.

12. Procédé selon l'une des revendications 1 à 11, **caractérisé en ce que** l'acide nucléique actif est un A.D.N. ou un A.R.N., un génome ou une partie d'un tel, comme par exemple un gène.

13. Procédé selon l'une des revendications 1 à 12, **caractérisé en ce que** l'acide nucléique actif présente une activité de modèle de l'acide nucléique et inclut une séquence pour la fixation de primer et de polymérase, l'acide nucléique étant amplifiable par un procédé d'amplification d'acide nucléique.

14. Procédé selon l'une des revendications 1 à 13, **caractérisé en ce que** l'acide nucléique actif provient d'une source eucaryote, telle une cellule de vertébré, une lignée de cellules tumorales, un oncogène ou un proto-oncogène, ou une lignée d'hybrides cellulaires.

15. Procédé selon l'une des revendications 1 à 14, **caractérisé en ce que** l'acide nucléique provient d'un micro-organisme, comme par exemple un protozoaire, une bactérie, un virus, un pathogène microbiologique ou moléculaire ou une partie de ceux-ci.

16. Procédé d'inactivation de virus, **caractérisé en ce qu'**à un matériau biologique contenant un virus est ajoutée une substance photodynamique choisie dans le groupe des phénothiazines, que le mélange est soumis à une irradiation par de la lumière laser avec une intensité de ≤ 0,1 J/cm² par cycle, pour diminuer l'activité de modèle de l'acide nucléique dans le matériau biologique d'au moins un facteur de 7 échelons logarithmiques, respectivement la teneur en acide nucléique jusqu'à < 100 pg/ml, de préférence < 10 pg/ml, encore mieux < 1 pg/ml, pour désintégrer ou pour inactiver pour l'essentiel le total de l'acide nucléique biologiquement actif dans le matériau biologique, tandis que l'intégrité et l'activité du matériau biologique reste essentiellement conservée.

17. Procédé selon la revendication 16, **caractérisé en ce que** le virus est un virus à coque de lipide, un virus à coque de non-lipide, un virus à A.D.N. ou à A.R.N. ou un virus atténué.

18. Procédé selon la revendication 17, **caractérisé en ce que** le virus, choisi dans le groupe des adénovirus, des herpès, des papoavirus, des poxvirus, des parvovirus, des réovirus, des myxovirus, des paramyxovirus, des picornavirus, des togavirus, des flavivirus, des orthomyxovirus ou des rhadovirus, est de préférence un virus de la grippe, un HIV, un HCV, un HAV, un HBV, un TBEV ou un CMV, ce virus pouvant être atténué le cas échéant.

19. Procédé d'inactivation de virus sans coque dans un matériau biologique protéinique, **caractérisé en ce qu'**une substance photodynamique, choisie dans le groupe des phénothiazines, est ajoutée à un matériau biologique dans une concentration d'au moins 20 µM et que le mélange est soumis à un faisceau laser dans le domaine de longueur d'ondes de l'activité maximale de la substance photodynamique avec une intensité de la lumière laser de ≤ 0,1 J/cm² par cycle, de préférence ≤ 0,06 J/cm² par cycle pendant au moins 20 cycles.

20. Procédé selon la revendication 19, **caractérisé en ce que** des virus sans coque sont inactivés, en particulier des virus du groupe des picornavirus et des parvovirus.

21. Procédé selon l'une des revendications 19 ou 20, **caractérisé en ce qu'**essentiellement le total de l'acide nucléique biologiquement actif et infectieux dans ce matériau biologique est désintégré ou inactivé, tandis que l'intégrité biologique et l'activité du matériau biologique protéinique reste conservée.

22. Procédé selon la revendication 21, **caractérisé en ce que** l'inactivation ou la désintégration de l'acide nucléique est contrôlée par un procédé de quantification de l'acide nucléique, et qu'un matériau biologique est obtenu, dont la teneur en acide nucléique est inférieure à 100 pg, de préférence inférieure à 10 pg, encore mieux inférieure à 1 pg par dose, respectivement présente au maximum 1 à 500 copies/ml, et qui est ainsi essentiellement exempt d'activité de modèle de l'acide nucléique.

23. Utilisation d'un procédé selon l'une des revendications 16 à 22 pour la fabrication d'un matériau biologique viralement inactivé, qui ne présente pas en particulier de virus infectieux à coque non lipidique.

24. Procédé selon l'une des revendications 16 à 22 pour la fabrication d'un vaccin inactivé contre un virus.

25. Procédé pour la fabrication d'un vaccin inactivé contre un virus, **caractérisé en ce qu'**à un matériau contenant un virus est ajoutée une substance photodynamique, choisie dans le groupe des phénothiazines, et que le mélange est soumis à un faisceau laser avec une intensité de la lumière laser de < 0,1 J/cm² par cycle, afin de désintégrer essentiellement le total de l'acide nucléique biologiquement actif, tandis que l'intégrité et l'activité biologique, comme l'antigénicité et l'immunogénicité du virus restent essentiellement conservées, **en ce que** la désintégration de l'acide nucléique est contrôlée par un procédé de quantification de l'acide nucléique et **en ce qu'**un matériau contenant le virus est obtenu, dont la teneur en acide nucléique est inférieure à 100 pg, de préférence inférieure à 10 pg, encore mieux inférieure à 1 pg par dose, respectivement présente au maximum 1 à 500 copies/ml, et est ainsi essentiellement exempt d'activité de modèle de l'acide nucléique, **en ce que** les virus inactivés sont soumis le cas échéant à une épuration supplémentaire et **en ce que** les virus inactivés sont formulés en un vaccin contre le virus avec un support physiologiquement acceptable.

26. Procédé selon la revendication 25, **caractérisé en ce que** le micro-organisme est un pathogène viral, tel un virus à coque de lipide ou de non-lipide, un virus porteur d'A.D.N. ou d'A.R.N., choisi dans le groupe des adénovirus, des herpès, des papoavirus, des poxvirus, des parvovirus, des réovirus, des myxovirus, des paramyxovirus, des picornavirus, des togavirus, des flavivirus, des orthomyxovirus ou des rhadovirus, est de préférence un virus de la grippe, un HIV, un HCV, un HAV, un HBV, un TBEV ou un CMV, ce virus pouvant être atténué le cas échéant.

27. Procédé selon l'une des revendications 25 ou 26, **caractérisé en ce que** le matériau contenant le virus provient d'une culture de cellules infectées par un virus, d'un reste de culture de cellules infectées par un virus, d'une composante de cellules infectées par un virus ou d'une solution épurée contenant des virus.

28. Procédé selon la revendication 27, **caractérisé en ce que** les cellules infectées par le virus sont des cellules de vertébrés, telles des cellules d'embryons de poulet, des cellules VERO, des cellules CV-1, des cellules LLC-MK-2, des cellules MDCK, des cellules MDBK, des cellules WI-38 OU des cellules MRC-5.

29. Procédé selon l'une des revendications 25 à 28, **caractérisé en ce que** l'acide nucléique inactivé ou désintégré provient de la cellule et / ou du virus.
